# EUROPEAN PATENT SPECIFICATION

(11) **EP 4 575 495 B1**
(45) Date of publication and mention of the grant of the patent: **24.09.2025**
(21) Application number: 23219985.1
(22) Date of filing: 22.12.2023
(51) Int. Cl.: G01N 33/497, G01N 33/00

(54) **METHOD AND SYSTEM FOR THE DETECTION AND/OR QUANTITATION OF MICROORGANISMS**
VERFAHREN UND SYSTEM ZUM NACHWEIS UND/ODER ZUR QUANTIFIZIERUNG VON MIKROORGANISMEN
PROCEDE ET SYSTEME DE DETECTION ET/OU DE QUANTIFICATION DE MICRO-ORGANISMES

(43) Date of publication of application: 25.06.2025
(73) Proprietor: Guerin, Joe, 85570 Markt Schwaben (DE)
(72) Inventor: Guerin, Joe, 85570 Markt Schwaben (DE)
(74) Representative: Prüfer & Partner mbB Patentanwälte · Rechtsanwälte

(56) References cited:
- CA-C- 2 204 121
- ALEXANDROS K PAVLOU: "Use of an electronic nose system for diagnoses of urinary tract infections", vol. 17, no. 10, 1 October 2002 (2002-10-01), Amsterdam , NL, pages 893 - 899, XP093157194, ISSN: 0956-5663, Retrieved from the Internet <URL:https://pdf.sciencedirectassets.com/271399/1-s2.0-S0956566300X01042/1-s2.0-S0956566302000787/main.pdf?X-Amz-Security-Token=IQoJb3JpZ2luX2VjELr//////////wEaCXVzLWVhc3QtMSJFMEMCH13AgWnr5zsiRO4YckIxidYqvCmH2frhfbng2yWUsCsCIGweOTufE6lfFaig0OW3+wbkguKATPvBCL5PV3Lu8bXvKrMFCBMQBRoMMDU5MDAzNTQ2ODY1IgwRxnfNJ> [retrieved on 20240429], DOI: 10.1016/S0956-5663(02)00078-7

## Description

### Field of Invention

This invention discloses an improved method for the detection of microorganisms. Detection is based on the ability of microorganisms to generate at least one volatile metabolic product, by catabolizing sulfur-bearing substances and thereby generating gaseous or vapor-phase sulfides as metabolic products.

### Background of the Invention

For over 100 years, the coliform test has served well as an indicator of the microbial quality of drinking water, recreational bathing water, and waters used for aquaculture. Although most coliform strains are benign to humans, their presence in water is usually a reliable indicator of pollution by sewage and of associated potential hazards posed by pathogens of human or animal origin. Originally defined by its ability to ferment lactose to produce gas and acid at 35°C, the coliform group comprises several genera, including *Citrobacter, Klebsiella, Enterobacter and Escherichia.* Following its widespread adaption, the coliform test was later refined to detect only organisms of fecal origin, i.e. coliforms normally found in the intestines of humans and warm-blooded animals. Fecal coliforms were defined as those coliforms with the ability to ferment lactose at a growth temperature of 44-45°C in a medium containing bile salts. Meanwhile, fecal coliforms are more generally referred to as *thermotolerant coliforms.*

Further advances in microbiological techniques allowed "total coliforms" to be more specifically defined as those members of the *Enterobacteriaceae* possessing the gene coding for the production of β-galactosidase, an enzyme which cleaves lactose into its constituent sugars glucose and galactose. Another enzyme, β-glucuronidase, is produced by approximately 94% of *Escherichia* strains. The detection of β-glucuronidase in a medium is now officially recognized as a reliable method of confirming the presence of *E.coli* and of distinguishing it from other coliforms. Most regulatory authorities, including the World Health Organisation, now regard *Escherichia coli* as the most reliable indicator of fecal pollution of drinking water. The detection of thermotolerant coliforms, in particular when confirmed by the indole test, is generally regarded as an acceptable alternative to the *E.coli* test for the monitoring of drinking water in low-resource countries.

Regulations in most developed countries do not tolerate any number of *E.coli* in drinking water. For example, EU Directive 2020/2184 on the quality of water intended for human consumption specifies 0 cfu/ 100ml. (0 cfu/ 250 ml for bottled water). Consequently, most official test procedures for the microbial testing of drinking water specify 100 ml as the minimum sample size necessary to capture any *E.coli* present in the sample. Moreover, any method used for the microbial testing of drinking water must be able to detect *E.coli* down to 1 cfu/ 100 ml of sample. Advantages and disadvantages of some common state-of-the-art methods for the detection and enumeration of fecal indicator organisms in drinking water are discussed below.

**ISO 9308-1:2014** - a Membrane Filtration (MF) method for detection and enumeration of *Escherichia coli* and coliform bacteria, entails filtering a 100 ml water sample through a membrane filter so as to retain bacteria on the membrane surface. The membrane is then placed face down on a chromogenic coliform agar plate and incubated at 36 ± 2°C for 21 ± 3 h. When acted upon by the enzymes β-galactosidase and β-glucuronidase, reagents specific to these enzymes release colored reaction products, enabling colonies of total coliforms and *E.coli* to be identified by their respective color and counted. An example of an improved, market-leading medium used for the enumeration of total coliforms and *E.coli* according to the MF method is m-ColiBlue24^{®} from Hach Company, Colorado, USA. Total coliforms and *E.coli* are grown on one single petri dish, then identified and enumerated according to the distinctive red and blue colors of their respective colonies. Incubation time is 24 h without the need for a confirmatory step.

A drawback of the MF method is the need to dilute water samples with anticipated high microbial counts in order to avoid overcrowding the cultivated agar plate. The method requires skilled labor to initiate the test and necessitates the presence of skilled personnel to finalize the analysis, including the counting of developed colonies with the aid of a microscope. As a further disadvantage, the method is not suitable for the testing of water that is turbid or contains suspended matter.

**Most Probable Number (MPN)** method for detection and enumeration of *Escherichia coli* and thermotolerant coliforms. Although this method has been largely replaced by ISO 9308-2:2012 (see below), the technique is still widely practiced. It involves enrichment of target organisms in test tubes of liquid broth and is therefore advantageous for waters containing particulate matter that could falsify the results of plate counting methods. Since the concentration of viable coliforms in a water sample is not known prior to the test, tenfold dilutions of the water sample are simultaneously incubated so that a useful working number of positive tubes (usually those producing gas) can be identified. In a typical test, three sets of tenfold dilutions, each containing five tubes, would be run for each water sample. In a typical presumptive test for total coliforms, the tubes are incubated at 37°C for 24 h. Tubes showing a negative reaction are incubated for a further 24 h. As a confirmatory test for thermotolerant coliforms, innoculate from coliform-positive tubes is further incubated in EC medium at 44 °C for 24 h to check for gas production. To test for *E.coli,* positive tubes from the presumptive coliform test are incubated in tryptone water at 44°C for 24 h. Kovacs' reagent is added to the incubated medium to check for the presence of indole. The number of positive tubes from each test and dilution is recorded. Statistical tables which account for the number of positive samples and their dilution factors are used to calculate the most probable number of total coliforms, thermotolerant coliforms or *E-coli* in the water sample. Conventional MPN methods are notoriously labor-intensive, typically requiring the preparation of 15 test tubes for each water sample, while the time to result can be as long as 72 h, depending on the level of confirmatory testing needed.

**ISO 9308-2:2012** is a Most Probable Number (MPN) method for detection and enumeration of *Escherichia coli* and coliform bacteria. This part of the ISO 9308 standard supersedes an earlier, conventional MPN method. It generally involves the use of proprietary *Colifert^{®}* Defined Substrate Technology^{®} and the Quanti-Tray^{®} system for enumerating total coliforms and *E.coli. Colifert^{®}, Defined Substrate Technology^{®}, DST^{®} and Quanti-Tray^{®}* are trademarks or registered trademarks of IDEXX Laboratories, Inc. or its affiliates in the United States and/or other countries. A chromogenic substance releases a yellow dye when acted upon by the enzyme β-galactosidase, thereby identifying a sample that contains one or more coliform colonies. A second, fluorogenic substance contained within the same reagent releases a fluorescent compound when acted upon by the enzyme β-glucuronidase. In this manner both total coliforms and *E.coli* can be enumerated in one single test.

In a typical analysis a 10ml, 100 ml, or 250 ml water sample is mixed with the proprietary IDEXX Colilert-18^{®}chromogenic/ fluorogenic reagent and poured into an appropriately-sized Quanti-Tray^{®} pouch. A special heated-roller instrument is used to seal the plastic pouch so that the water sample is partitioned between 51 wells (Quanti-Tray^{®}) or 97 wells (Quanti-Tray^{®}/2000). To test for total coliforms and *E.coli* the tray is incubated at 35 ±0.5°C for 18 h (maximum 22 h). To test for fecal coliforms the tray is incubated at 44.5 ±0.5°C for 18 h (maximum 22 h). At the end of the incubation period the tray is visually inspected. The yellow wells are counted and recorded as positive for total coliforms. The tray is then examined under UV light to identify any fluorescent wells. These are counted and recorded as positive for *E.coli.* Statistical tables that account for the number of positive wells are used to calculate the most probable number of total coliforms, fecal coliforms or *E-coli* in the water sample. The system detects down to 1 organism/ 100 ml and can measure counts up to 2,419 with Quanti-Tray^{®}/2000.

Compared with conventional MPN methods that are slow, labor-intensive and require high skill levels, the Quanti-Tray^{®} method is a reliable, easy to use method that introduced a major improvement to the state of the art. Despite the reduced level of skill needed to initiate the water analysis, the method requires the attention of experienced staff within the relatively narrow time window between 18 and 22 h to count the positive wells, to correctly identify the fluorescent wells, and to finalize and document the analysis. While the use of an enzyme-specific fluorogen allows measurement and detection of *E.coli* with a confidence level of 95%, methods that rely on β-glucuronidase cannot detect harmful enteric bacteria such as *Clostridium perfrigens* or *Salmonella.* A further consideration is the negative environmental impact associated with the disposable plastic tray used for each water sample.

**ISO 9308-3** is a miniaturized Most Probable Number (MPN) method for detection and enumeration of *Escherichia coli* in surface and waste water, using a liquid medium. The water sample may be diluted up to 1/ 200000, allowing measurement of highly polluted waste water having *E.coli* counts in the 10E+08 cfu/ 100 ml range. A 200 µl aliquot of each pre-diluted sample is added to one of 96 wells of a microtitre plate that contains a fluorogenic reagent. Following incubation of the plate at 44 ±0.5°C for a minimum of 36 h, wells which are fluorescent when viewed under UV light are considered positive for *E.coli.* MPN tables are used to enumerate the population of *E.coli* in the water sample.

The method is advantageous for water samples containing particulate matter, as well as for water from polluted sources. On account of its relatively small sample size the method is not suitable for drinking water and any other type of water for which the guideline is less than 15 counts per 100 ml. In a similar way to other MPN techniques, the method is labor-intensive and requires the attention of experienced staff at the end of the experiment to count the number of positive wells, including the correct identification of fluorescing wells.

### Immunological, PCR, DNA and other Biochemical Methods

Test methods that rely on incubation bear the disadvantage of long waiting times for the test result. As a consequence, much research is currently focussed on developing laboratory procedures and test kits which employ PCR sequencing, the use of antibodies, oligonucleotides, etc to deliver faster detection or enumeration of fecal coliforms.

An advantage of quantitative polymerase chain reaction, (qPCR) and other advanced technologies is their ability to unequivocally identify the target bacterial strain, often based on detection of a unique gene sequence. Such molecular techniques typically work with droplets, sometimes microliter volumes, of test medium and usually necessitate a preparatory concentration step such as membrane filtration. A further challenge of DNA-based methods is to distinguish between living and dead organisms, to avoid overestimating the active population. PCT patent no. WO 2020/021010 A1 describes a method for detection or quantitation of *E.coli* and *Enterococcus spp.,* based on amplification and measurement of bacteria-specific nucleotide sequences. Results are achieved within four hours. Following membrane filtration of the water sample, its RNA content is first extracted to confine the ensuing analysis steps to living/ viable organisms. Other research in this field involves the use of antibody-functionalised magnetic nanoparticles to which the target organisms bind themselves, allowing viable cells to be magnetically separated for subsequent analysis.

In general, test methods based on advanced microbiological and biochemical test methods require at least some access to laboratory equipment and sophisticated instrumentation for sample preparation and analysis. Microfluidic and lateral flow devices based on such techniques offer the promise of faster times to detection in comparison with methods requiring test sample cultivation. However, to date such devices typically lack sensitivity and are relatively expensive.

### Automated/ Signaling Methods

The membrane filtration and MPN methods described above require the presence of a skilled analyst at the end of the incubation period to finalize the analysis, count the number of formed colonies/ positive tubes/ wells, interpret the data, and document the test result. Most test protocols prescribe a minimum incubation period of 18, 24, 36 h, etc, before results can be evaluated. These factors place a considerable restriction on test scheduling. As one example, a water sample received late on Friday afternoon may require specialist laboratory work on Saturday evening to finalise the analysis.

A number of automated instruments are now commercially available that overcome the restrictions associated with manual evaluation of test results. A key advantage is that the test result is made available once a threshold bacterial count has been detected. For polluted samples, automated measurement can shorten analysis times by several hours compared with manual methods that prescribe a minimum incubation period, regardless of contamination level.

The XplOrer64^{™} is an automated instrument and method from the company Bio-Rad Laboratories, Inc (Hercules, California, USA). It uses impedance measurements in a liquid medium to track the growth of a bacterial population over time. Specific liquid culture media are available for *E.coli, Enterococci* and coliforms. Metabolism of the growth media by the target organism causes changes in impedance that are monitored by the instrument. The measurement electrodes and proprietary liquid medium are contained in pre-prepared, single-use 10 ml vessels. 100 ml of water sample is first filtered, the filter membrane is then immersed in the culture medium within the 10 ml vessel. The instrument, which can accomodate up to 64 test samples, allows real-time monitoring of bacterial growth and transmission of a test report via a LIMS interface.

The Tecta^{™} Automated Microbiology Detection System from the company IDEXX Laboratories, Inc. is an EPA-approved test method and instrument for the automated quantitation of *E.coli, Enterococci* and coliforms. The water sample is filled into a 100 ml cartridge without the requirement for any prior preparation. The cartridge contains dry enzyme substrate medium specific to the target organism, (e.g. a fluorogen which is sensitive to β-glucuronidase for detection of *E.coli*). A change in color/ fluorescence of the liquid medium is detected via a patented optical coupling at the base of the 100 ml vessel. The system detects down to 1 organism/ 100 ml with automatic transmission of the test report by e-mail to the analyst.

Automated test methods can cover a wide dynamic range up to 10E+09 cfu/ 100 ml without the need for pre-dilution of samples. Reproducibility is improved since there is no reliance on subjective interpretation of results. Staff overtime and shift work needed to finalise and document multiple analyses can potentially be reduced. The single-use test vessels and proprietary culture medium used in automated instruments can cost $15 - $20 per water sample, plus shipping costs. In addition to the relatively high cost of consumables, the creation of plastic waste with every water sample tested is undesirable from an environmental perspective.

### Presence/ Absence (P/A) Methods

In contrast to test methods that quantitate a bacterial population, P/A methods return a positive result if one or more organisms are detected in the test sample. A negative result signifies that the test sample is free of bacteria. P/A methods do not reveal the degree of pollution. They are particularly useful for applications where zero coliforms are expected or permitted, such as when testing for compliance with the EPA Safe Drinking Water Act. Readycult^{®} Coliforms 100 (Merck KGaA, Darmstadt, Germany) is a widely used P/A test for coliforms and *E.coli.* The dry medium is added to a 100 ml water sample, which is incubated at 35-37°C for 24h. A chromogen (X-Gal) that is sensitive to the enzyme β-galactosidase is used for coliform detection, while the presence of *E.coli* is indicated by the action of β-glucuronidase on the fluorogen MUG. The formulation also contains tryptophan so that the incubated liquid can additionally be tested for indole, should a further confirmatory test be required.

### The Hydrogen Sulfide (H₂S) Test

Since the early 20^{th} century the Hydrogen Sulfide test has been used for the biochemical detection of fecal indicator and other bacteria that generate H₂S as a metabolic product. Compounds of sulfur such as sulfates, thiosulfates, sulfites, or organic sources such as cysteine were added to the test medium as precursors of H₂S. Soluble salts of heavy metal ions such as Fe²⁺ or Pb²⁺, either added to the culture medium or suspended above it as impregnated paper discs, formed black sulfides on contact with H₂S, thus revealing the presence of sulfide producing bacteria. [see "Hydrogen Sulphide Production by Bacteria", Clarke, P.H. (1953), J. Gen. Microbiol. 8, 397-407].

Today, an extensive range of commercially available liquid and agar culture media employs the same technique for the biochemical identification of microorganisms that catabolize sulfur-bearing molecules to produce sulfide reaction products. One of very many examples of such culture media is Bismuth Sulfite Agar, which is used for selective identification and isolation of *Salmonella spp.* It contains enzymatic digests of casein and animal tissue as sources of carbon, nitrogen and vitamins. Bismuth sulfite and brilliant green inhibit growth of Gram-positive bacteria and coliforms. Ferrous sulfate acts as both a source of sulfur and as an indicator, producing brown to black iron sulfide precipitate in the cultivation medium to identify colonies of *Salmonella.*

The United Nations 2023 update on Sustainable Development Goal 6 states that 2.2 billion people still lacked safely managed drinking water services, 3.4 billion lacked safely managed sanitation services, and 1.9 billion lacked basic hygiene services in 2022. The report states that fecally derived pathogens are the principal concerns in setting health-based targets for microbial safety. The Hydrogen Sulfide test, as used for the surveillance of microbial water quality, is widely accepted and used as a presence/absence test in developing and low-resource countries. Its use and advantages were first published by Manja et al following a comprehensive comparison of the method with a conventional MPN technique, based on analyses of 699 water samples from sources throughout India; [Manja et al: "A simple field test for the detection of faecal pollution in water", Bulletin of the World Health Organisation, 60 (5): 797-801 (1982*)].* The formulation developed by Manja was prepared as a concentrate, consisting of: 20 g of peptone (growth medium); 1.5 g dipotassium hydrogenphosphate (buffer); 1 g sodium thiosulfate (sulfide precursor); 0.75 g ferric ammonium citrate (H₂S indicator); 1 ml Teepol (selective agent); 50 ml water. 1 ml of this concentrate was dried on a paper strip, sterilized, and placed in a sterile bottle. 20 ml of the test water sample were added to the bottle, which was held at ambient temperature (30-37°C). Samples that developed a black coloration after 12-18 h were recorded as positive for fecal pollution. H₂S test kits are meanwhile available from major laboratory suppliers, but are also manufactured and sold locally in developing countries. They are available as pre-prepared bottles, usually containing the detection medium in solid form or impregnated on a paper or woolen substrate. Alternatively, the H₂S test is sold as a powder in a sterile sachet for mixing with the test water sample.

Many studies have been conducted to evaluate the suitability of the H₂S P/A test as a viable, less expensive alternative to established test methods. Most studies found very good correlation, typically 80-90%, between the results produced by the H₂S test and those of officially approved methods.

Several studies have commended the H₂S test on its ability to detect potentially hazardous bacteria that do not belong to the coliform group but are nevertheless associated with the gastrointentestinal tract of warm-blooded animals.

The UNICEF Target Product Profile, "Rapid Water Quality Detection Tests", "Products currently used by UNICEF for E.coli testing", Ver. 4, Jan. 2023 summarises the H₂S test as follows: "H₂S Presence/ Absence Test: This is a simple method for detecting the presence of hydrogen sulphide producing bacteria (an alternative indicator of faecal contamination); these tests cannot quantify the magnitude of contamination. This method does not require technical training and requires an 18-24 hour incubation period (though it can be done at room/body temperature). This test can yield false positives, depending on the type of water source, due to the natural presence of these bacteria in tropical soils. Tests are one-time use. Approximate cost: US$0.50 - US$2 per test."

### The "electronic nose" system

Pavlou et al., "Use of an electric nose system for diagnosis of urinary tract infections", Biosensors and Bioelectronics, vol. 17 no. 10, pp. 893-899 (2002) describes the use of an array of "electronic nose" sensors to evaluate mixtures of volatile substances taken from the headspace of incubated urine samples. The electronic nose sensors employ arrays of conducting polymer sensors to characterize the composition of gas and vapor samples. Volatile substances, often large molecules characteristic of a certain odor, interact with the conducting polymer to alter its electrical conductivity. The electronic nose employs complex pattern recognition algorithms, including multivariate statistical analysis and neural networks, to resolve the complex signal output from the numerous conductive polymers.

### Summary of the Invention

An object of this invention is to improve upon current state-of-the-art methods, as used in both low resource and more developed countries, to allow faster time-to-result, less sample handling and operator intervention, lower cost of consumables, and a reduced environmental impact.

For solving the object, the present invention provides a method as set forth in claim 1, and a system as set forth in claim 6. Preferred embodiments are set forth in sub-claims to claims 1 and 6, respectively.

The present invention thus provides an improved method and an improved system for detecting and/or quantitating microorganisms. The methods and systems according to the present invention allow a faster time-to-result, less sample handling and operator intervention. It provides methods and systems at lower cost of consumables, and a reduced environmental impact. The methods and systems according to the present invention can be readily automated to determine, if desired quantitatively, a bacterial population in a test sample or test medium. The test result may be transmitted, e.g. by electronic means, to a remote user, an analyst or other interested party. The methods and systems can be embodied as particularly useful and advantageous versions of the hydrogen sulfide test with the potential to make a significant contribution to the affordable screen testing of water sources and early detection of waterborne pathogens. This is of particular interest and value in developing countries. The present invention enables the detection of *Escherichia* coliforms with a high degree of confidence, based on improved culture media, more sensitive detection of sulfides (in particular H₂S), and by allowing for rapid confirmatory tests. Accordingly, it is well suited to the detection of sulfide producing bacteria on various medical, agricultural, environmental or industrial applications.

### Detailed description of the Invention

This invention discloses a method and a system for the detection and/or quantitation of microorganisms that generate at least one volatile metabolic product. In case of quantitation, the population/concentration/amount of a certain microorganism of interest in a given test sample or test medium is determined. In the present disclosure, this concept of quantitation is generally and commonly referred as quantitating the population of a microorganism.

The present invention applies the metabolization of sulfur-bearing molecules to generate hydrogen sulfide (H₂S).

The presence of H₂S is established on the basis of its accumulation within a confined space, without the need for conventional gas sensing. Many gas-sensing technologies are known, each with its own strengths for a specific safety, environmental or industrial application. Some of the many sensing and detection principles include: electrochemical, metal oxide, pellistor, photometric, infrared, and conductive polymer. Typical for such gas sensors and detectors is the desirability of a low detection limit, precise and reproducible conversion of a measured physical quantity into an accurate readout of analyte gas concentration or the issuance of a warning at a given threshold gas concentration, fast response time, and a wide dynamic measuring range. It is important to differentiate the functional principle of the detecting method used in this invention, which does not rely on the measurement of gas concentration as such, from that of state-of-the-art gas sensors, gas detectors, and gas analyzers. To achieve the objects of this invention it is not necessary to measure the concentration of a gas. More specifically, it is not necessary to monitor the concentration of a gaseous metabolite, as produced during the incubation of a test culture, within a dynamic measuring range, over the course of a microbial test. Instead, the detecting principle applied in the present invention is based on the unidirectional change in a property selected from an electrical property, a magnetic property, and an electromagnetic property, of the gas-detecting-device (for instance an electrical resistance) in response to the accumulation of H₂S in a confined space or volume. The method and system of the present invention is only required to detect the surge of H₂S generated by the exponential growth of a microorganism, when present, within a confined space, or volume that is in communication with the culture medium. Accordingly, the device has the character of a fuse, as opposed to the desired attributes of a sensor, as described above. For the purpose of this invention, the gas-detecting device will sometimes be referred to as a "gas-detecting fuse". By way of example, further illustration and differentiation, a sensor for NH₃ that responded to the accumulation of NH₃ over time would not be permissible for worker safety monitoring, since ongoing exposure to low-level background concentrations of NH₃ would eventually generate a false alarm. A smoke detector that continued to sound alarm after the room had been cleared of smoke would also not fulfil its intended purpose. Consequently, there has been little or no market interest in developing gas-detecting devices that respond to an accumulated dose of gas, and such products are rare or generally unknown.

The change of the electrical, the magnetic, and/or the electromagnetic property manifests itself as an irreversible change during the process of the test. This, however, in no way rules out the re-use of the gas-detecting device. More specifically, as distinct from continuous sensing within a dynamic range, the concept of the present invention is based on the detection of an abrupt, unidirectional change. Such an abrupt change is observed as a sudden decrease in the resistance of a gas-detecting film in an embodiment of the present invention, when contacted by a gaseous sulfide metabolic product, and refers to the behaviour of the gas-detecting film during the course of an incubation measurement. After completion of said measurement and exposure of the gas-detecting device to air, ohmic resistance of the gas-detecting film may return, or is treated to make it recover, to a higher ohmic value. If desired, the gas-detecting device may therefore be designed as a device that is suitable for re-use in several water tests, in spite of the abrupt decrease in resistance observed during the course of a water-testing procedure.

The method of the present disclosure is typically designed as an *in vitro* method.

The present disclosure describes the use of a gas-detecting fuse in combination with a culture medium as a novel test method for the detection of H₂S-producing microorganisms. The gas-detecting fuse may use a chemiresistive film that irreversibly alters its resistance when exposed to H₂S or other sulfides. Several sensor types such as conductive polymer and metal oxide rely on chemiresistance for chemical sensing. However, such sensors are invariably designed to have favorable hysteresis characteristics to maximise linearity and reproducibility, as usually required to output a signal proportional to the measured gas concentration. Consequently, they require a much higher level of sophistication than that of the present invention. For example, to achieve a linear response, metal oxide sensors are typically heated to 200°C, while nearly all gas sensors require initial factory or routine calibration. For these reasons it has until now not been obvious to those skilled in the art that a rudimentary sensing film could enable the objects of this invention.

The advantages of using a simple gas-detecting fuse to solve the object of this invention are manifold: (a) It can be positioned in direct contact with the vapor space above the liquid culture medium, at elevated temperatures, over incubation periods of several days if necessary. Commercially available gas sensors would find such harsh conditions extremely challenging or impossible. (b) The device is not cross-sensitive to fermentation by-products such as CO₂, alcohols, alkanes, amines, esters, etc. which would typically be generated in the course of bacterial growth. Market-available gas sensors would generate false alarms or lose their functionality if exposed to such environments over extended monitoring periods. (c) The solid chemiresistive film is largely insensitive to fluctuations in pressure. This feature allows the gas-detecting fuse to be positioned in vessels containing compressed gas. Electrochemical gas sensors used for H₂S sensing commonly incorporate a membrane diaphragm that would likely warp, generating false alarms if placed in contact with the headspace above a culture medium where fermentation gases typically cause a build-up of pressure. (d) The gas-detecting fuse could be mass-produced as a low cost printed sensor or adhesive tag, making it ideal for the frequent screening of large numbers of water samples or use in low-resource countries. (e) Its low cost and miniature size render it ideal for use as single-use sensor, while also minimizing its environmental impact. A disposable sensor is to be preferred for reasons of hygiene, given the proximity of the sensor to a potentially enriched culture of bacteria. Alternative designs based on a gas sensor, gas sampling lines, fiber optic bundle, etc., which were placed in contact with the test culture, would require frequent cleaning and sterilization. (f) The device is only required to signal the emergence of H₂S, not to measure its concentration. This obviates the need for calibration, thereby saving fabrication costs and facilitating operation by non-skilled staff. (g) Unlike chemiresistive sensors, the functionality of the gas-detecting fuse is not unduly sensitive to slight variations in film thickness or film morphology, and therefore does not require sophisticated and expensive fabrication technologies. (h) The detection of H₂S via a change in the resistance of a film requires only simple, low-power electronics, the basis for a favorably priced instrument suitable for mobile use in the field.

The ability to detect the presence of H₂S from within the vapor space above the test culture medium has two very distinct advantages over methods where electrodes, optical sensors, or other probes are immersed in the test medium: (a) Measurement is not prone to falsification arising from turbidity, the presence of particulate matter, or coloration of the test liquid. Fouling of electrodes or of optical windows is avoided. Many reagents that are added to culture media for the selective growth of target organisms, or as pH indicators, impart a deep color to the test sample which could create challenges for the use of optical detection methods. A few examples of colored reagents are brilliant green, crystal violet and bromothymol blue. Microbial test methods based on the color change of enzymatic/ chromogenic reagents added to the test medium would be ill-suited for the direct testing of opaque or turbid clinical samples such as blood or urine, which might otherwise require the use of agar plating techniques. (b) Measurement is essentially independent of the test sample volume. The gas-detecting fuse described in this invention can be applied to test sample sizes ranging from a few milliliters up to several liters. The ability of the invention to quantitate bacterial populations within a dynamic range from 10E+00 up to 10E+08 cfu/ 100 ml is therefore possible without preparatory sample dilution steps and is independent of the sample size. This is a distinct advantage over test methods that use immersed probes, where each test volume size would require its own test vessel with corresponding electrode spacings, optical pathways, etc. For the same reasons the present invention also has advantages over MPN methods that require pre-dilution of water samples with anticipated high bacterial densities. The present invention therefore enables the direct testing of 100 ml and larger water samples, as recommended by US and European guidelines for the testing of drinking water. At the same time it enables both the use of small sample sizes to reduce the cost of added reagents, or larger sample sizes for higher accuracy of measurement. Furthermore, the method described in this invention can include a pre-filtration step to reduce time-to-detection and achieve faster results. Microorganisms from a large water sample are collected on a membrane filter that can then be incubated in a small test volume until sulfide detection.

In an embodiment, the invention may involve the steps of:
a) Preparing a gas-detecting device, such as a gas-detecting fuse, a dosimeter, a sensor or other detecting device whose electrical or magnetic or electromagnetic properties are altered when contacted by a sulfide compound;
b) Preparing an electronic assembly or measuring instrument that is capable of detecting changes in the aforementioned electrical, magnetic, or electromagnetic properties of the detecting device, thereby generating a signal when a pre-selected threshold value is crossed, and transmitting the result via e-mail, sms or other means to a remote instrument or remote user/party;
c) Preparing a growth medium selective to the target microorganisms which contains a sulfur-bearing substance or substances;
d) Transferring a test sample of water or other medium to a sterile, sealable vessel;
e) Adding a defined quantity of the said cultivation/detection medium to the test sample and mixing;
f) Attaching the gas detecting device to said vessel so that the sensing region of the detecting device is in contact with the vapor/gas phase above or adjacent to or in communication with the test/culture medium;
g) Sealing the vessel;
h) Placing the sealed vessel with said attached gas detecting device into an incubator, instrument, or environment suitable for the cultivation of the target microorganisms;
i) Connecting the gas detecting device, via electrical connectors or by means of wireless transmission, to said electronic assembly or measuring instrument,
j) Setting the incubating device to the desired incubation temperature and starting incubation;
k) Pressing a "START TEST" button, or conducting a suitable action, to record the time at which incubation was started.

The measuring instrument, which when measuring the change of an electrical property comprises a suitably electronic assembly, records the time elapsed from the start of incubation until the emergence of volatile/ gaseous sulfide within the vapor or headspace of the cultured medium. The kinetics of bacterial growth is well understood within the field of microbiology. Following a lag phase in which the microorganisms adjust their metabolism to the prevailing conditions, the culture enters the log phase in which the number of cells doubles within a generation time characteristic to the strain of bacteria, thereby generating a surge in the quantity of sulfide produced by the bacterial population. Experiments have shown that, at a constant incubation temperature, there is a linear relationship between the logarithm of the initial bacterial population and the time to sulfide detection using the concept of the present invention. The present invention therefore allows the bacterial population of a water sample under test to be automatically computed from a calibration curve of log N₀ Vs t_{d}, where N₀ is the number of organisms present at the beginning of the incubation period and t_{d} is the time elapsed until sulfide detection.

A simple presence/ absence H₂S test is used extensively throughout the developing world as a low cost method of screening for the fecal pollution of drinking water. The test sample is typically incubated at ambient temperature without the use of an incubator. Depending on the test protocol, the test bottle is checked at intervals of 12, 18, 24, 36, 48, or 72 h for signs of blackening of the test medium. A positive result can only be detected after sufficient sulfide precipitate has formed to be visible to the naked eye. Visually recognizing a color change is challenging for small (e.g. 5 ml) test volumes, or in instances where the water sample is turbid or colored.

By contrast, the automatable test method of the present invention is sensitive to microgramm amounts of sulfide/H₂S, enabling early detection that does not rely on subjective human interpretation. Surprisingly, experiments have shown the test method to yield very precise results. Duplicate or triplicate test vessels of the same water sample yield time-to-detection signals that arrive within a few minutes of each other, even after several hours of incubation. A bacterial population in the 10E+08 cfu/ 100 ml range will yield a result after approximately 3 hours. Populations down to 1 cfu/ 100 ml are detected after approximately 11 hours. These analysis times compare favorably with commercially available automated methods.

Test samples that are free of target microorganisms are identified as such by the absence of a detection signal within a time limit specific to the individual bacterial species. A possible concern with respect to the detection of H₂S as a method to test for microorganisms in a medium is that sulfides present in the water sample from non-biological sources could potentially lead to a false positive test result. Such concerns are unfounded with respect to the present invention, since the presence of an initial inorganic or organic sulfide concentration in the test sample would be quickly detected, thereby invalidating the test. If necessary in such instances, measures could be taken to remove or neutralize the residual sulfide prior to microbial testing.

The gas-detecting device disclosed in one embodiment of the present invention uses the formation of an electrically conductive metal sulfide for the detection of hydrogen sulfide or other sulfide products of bacterial activity. The precipitation of a dissolved metal ion as its water-insoluble sulfide is a well-established practice in qualitative analytical chemistry. In a manner similar to other elements, especially those of Groups V and VI of the periodic table, the sulfide ion forms very many compounds that display good electrical conductivity. While many elements react with the sulfide ion to form conductive sulfides, the present invention uses copper in the form of copper(I) oxide, "cuprous oxide", "Cu₂O", as the preferred reaction partner for H₂S. Copper(I) oxide is practically insoluble in water and therefore well suited for deployment in environments having high relative humidity.

The electrical resistance of the chemiresistive copper(I) oxide film, as used for the detection of H₂S in an embodiment of the present invention, typically lies in a range from 1 Megaohm to 1000 Megaohm (MΩ) or from 20 Megaohm to 750 Megaohm (MΩ). As outlined above, since the gas-detecting device of this invention is not used for gas sensing in the conventional sense, there is no requirement to correlate a range of electrical resistance to a calibrated range of gas concentration. As observed in the course of this invention, the emergence of H₂S from a water sample under cultivation results in a very sharp decrease in resistance, typically by five or six orders of magnitude, to end resistance values of between 10 and 5000 Ohm (Ω). The low resistance of the sulfide film is particularly advantageous to the invention, since it lies well below the resistance imparted to the test electrodes by the presence of water and other vapors in the headspace of the test medium that might otherwise impair detection.

The intrinsic chemical stability and favorable electrical properties of the copper(I) oxide film enable the gas-detecting device (H₂S-detecting fuse) of the present invention to be constructed as a very simple device. For the purpose of demonstrating the concept of the present invention, a paste consisting only of copper(I) oxide and a binder is applied as a spot between a pair of electrodes. A few mg of this inexpensive, non-toxic chemiresistive paste is sufficient for the manufacture of 100 H₂S-detecting fuses. A 6-pin, 0.5mm pitch, flat flexible (FFC) cable serves to provide three parallel pairs of electrodes having individual electrode spacings of approximately 250 microns. A spot of the paste is applied between each of the three pairs of pins to create three resistances in parallel. The film is disposed as three parallel resistances for reasons of redundancy and reliability only; this configuration is not necessitated by detection requirements. A change in the resistance of the chemiresistive film is detected by a simple DC test circuit. In the apparatus used to validate this invention, the chemiresistive film is placed in series with a reference resistor in a circuit to which a DC voltage of 80 mV is applied. The voltage across the reference resistor is monitored. The action of H₂S on the chemiresistor film causes a sharp decrease in the resistance of the latter, which, in turn, induces an increase in the voltage measured across the reference resistor. In the apparatus used to validate this invention the voltage across the reference resistor is monitored by a datalogger with PC visualization software. During a typical water testing experiment the output signal from the DC circuit is observed as a flat baseline, corresponding to a high ohmic resistance of the chemiresistive film. The emergence of H₂S within a given test vessel is observed as a steep rise in the measured voltage as measured by the datalogger software. When the voltage signal exceeds a setpoint indicating that the resistance of the chemiresistive film has fallen below 3 kΩ, the datalogger initiates a program that computes the time interval from the beginning of incubation until detection of H₂S. The software program calculates the bacterial population originally present in the test sample from the sample's time-to-detection. This test result is automatically forwarded to the analyst by e-mail. While the use of a datalogger has been useful to demonstrate and validate the test method, it will be obvious to those skilled in the art of electronics and instrumentation that the detection in a change of resistance, computation of time-to-detection and bacterial population, as well as the transmission of the test result to the analyst, could readily be achieved by more rudimentary electronic circuitry.

Although the present invention has been described in detail with reference to the foregoing embodiments, those skilled in the art will appreciate that the technical solutions of the above-mentioned embodiments can well be modified, or some or all of the technical features thereof can be modified or varied or equivalently replaced; these modifications or replacements do not cause the essence of the corresponding technical solutions to deviate from the scope of the technical solutions of the embodiments of the present invention. For example, numerous material regimes could be chosen for the chemiresistive detection of H₂S, which include but are not limited to metals or their alloys, non-metals, metallic and non-metallic compounds, composite materials, conductive polymers, nanoparticles, carbon nanotubes, nanofibers, nanowires, 2-D materials, graphene, quantum dots, or self-assembled monolayers. Further, binders of various sorts and kinds may be used to achieve similar or improved mechanical and electrical properties of the gas-detecting film. Also, changes to any other electrical property and/or a magnetic property (which may also include an optical property or an electromagnetic property) of a material arising from contact with a sulfide could readily be used to construct a similar or equivalent device for the detection of sulfide as disclosed here. Such electrical and/or magnetic (optionally including optical or electromagnetic) properties include, without being limited to, electrical resistance, impedance, conductivity, capacitance, inductance, electrochemical, dielectric, frequency-related, or any electrical or magnetic property or quantity to which an SI unit has been assigned. It will further be obvious to those skilled in the art that the simple method disclosed here for the measurement of resistance could be replaced or improved upon by the use of alternative DC and AC measurement techniques, including but not limited to: lower or higher test voltages, amperometric methods, impedance measurement, frequency-related methods, voltammetry, or polarography. It will also be obvious to those skilled in the art that mechanisms differing from those disclosed within the scope of this invention can be exploited to induce changes to the electrical or magnetic properties of a material upon its exposure to H₂S. These include, but are not limited to: sulfide doping, changes in the electrical resistance of a conductive polymer, for instance based on the acidity of H₂S, changes in the magnetic properties of a ferro-, ferrimagnetic, or other magnetic material, for instance based on the influence of sulfide on grain boundaries or surface properties. It will further be obvious to those skilled in the art that numerous measures could be implemented to decrease the final resistance of the chemiresistor film, or to increase or decrease its sensitivity or response time, if so needed to realize a desired embodiment of this invention. Such measures include, but are not limited to: altering the spacing between the electrodes, placing resistances in parallel with the measuring electrode(s); the use of interdigited electrodes or other electrode configurations; the use of alternative electrode materials; the use of alternative electrode substrates; modifying electrode geometries, or the contact boundary between electrode and chemiresistive film; the addition of metallic or otherwise conductive particles to increase the number of conduction pathways throughout the detecting film; altering the film porosity; the use of catalyst substances; the incorporation of the sulfide detecting area into a sensitive region of an amplifying semiconductor device, for example the gate or base region of a transistor. It will also be obvious to those skilled in the art that the chemiresistive film described in the present invention can be fabricated and improved upon using any of many established or novel deposition or printing techniques, including but not limited to: photolithography, evaporation, sputtering, chemical vapor deposition, physical vapor deposition, atomic layer deposition, epitaxial growth, MEMS technology, organic semiconductors, printed electronics, conductive inks, spin-on coating, drop-casting, spraying, painting, inkjet printing, molecular imprinting, or other printing techniques.

If in the presently disclosed specific embodiment a chemiresistive film is to be applied manually to the electrodes in the form of a paste, any suitable water-resistant binder that imparts good mechanical strength to the film can be employed. A sharper, more discrete detection signal is achieved if a reactive binder is chosen which promotes covalent linkages across the chemiresistive film. For this purpose a suitable carboxylic acid or powderized metal may be used. In one embodiment, 60 microliter of a 0.0025 molar aqueous citric acid solution is added to an agate mortar. 50 milligramm copper(I) oxide is added. The mixture is ground with a pestle until a smooth paste forms. The paste is ground further until the formation of bubbles is observed. The frothing paste is ground for a further three minutes. A ceramic ball mill or other suitable grinding method may alternatively be used. A suitable precision writing utensil may be used to apply the paste to the electrodes. For instance, a 0.05 mm fineliner felt-tip pen, free of any ink, may be used for this purpose. After application of the film, the electrodes are dried at 70°C for 30 minutes.

In a particularly advantageous embodiment the change to the resistance of the chemiresistive film is transmitted by a wireless method to a nearby measuring instrument, without the requirement of a cable connection between the test vessel and the measuring instrument. This allows the test samples to be placed into an existing incubator, without the requirement that the measuring instrument must necessarily possess an incubating function. In this manner a large number of test samples are simultaneously incubated using an existing item of laboratory inventory (an air fan- or water bath incubator), while using a single measuring instrument positioned adjacent to the incubator to detect H₂S-positive samples and transmit the results by e-mail or other electronic means to the analyst. For example, a low-cost, passive RFID tag, which was presented by a group at the University of Waterloo *[*Challenge: RFID Hacking for Fun and Profit, Ju Wang et al, MobiCom'18, October 29-November 2, 2018, New Delhi, India], can easily be modified for use as sensors. Examples are provided to describe RFID tags modified to enable the wireless measurement of temperature, light, gesture, pressure, etc. The method can be applied to the measurement of any environmental variable that is transduced by the sensor into a variable resistance or impedance. A small section of the tag's antenna is removed and replaced by the variable resistor of the individual sensor. The technique measures a variable over a certain range and even the simplest sensor described detects the bistable states of, "door open" and "door closed". Again, the same principle of irreversible change as described above can be applied to distinguish a sensor designed to monitor a given parameter range or bistable state from a simple fuse-like detection device. The present invention makes use of the time to detection of H₂S as the basis for computing the bacterial population, not directly measuring the concentration of H₂S as such. The chemiresistive film used in the specific embodiment, or other electrical or magnetic film or sensing entity, can be deposited as a coating on the antenna of an RFID tag so as to irreversibly alter the signal received by the reader, thereby signalling the detection of H₂S. Or more simply, the chemiresistive film or other sensing entity is connected so as to short-circuit electrical contacts on an RFID tag, thereby signaling the detection of H₂S to the reader. Such a design also allows the chemiresistor to be positioned inside the test vessel, electrically connected via a simple feedthrough connector to an RFID tag affixed to the cap of the vessel. The use of RFID technology for the wireless monitoring of test samples also allows the ID of each test sample to be encoded in the RFID tag. This is particularly advantageous in instances where a large number of water samples are to be tested, tracked and documented. Obviously, the method described here for RFID could also be applied to NFC, WLAN, or numerous other wireless transmission protocols.

An aspect of the present invention is the provision of a novel, signaling method for the detection and quantitation of fecal indicator bacteria (FIB) in drinking and recreational water, while also facilitating confirmatory testing for *Escherichia coli* and thermotolerant coliforms. For this purpose, a cultivation and detection medium (CDM) has been adapted to the methods and systems of the present invention, wherein the CDM is mixed with the test water sample to promote the selective growth and enable detection of the aforementioned organisms.

In order for the CDM to allow the generation of at least one volatile metabolic product by the microorganism, a sulfur-containing substance is added to the medium in a preferred embodiment. For a conventional H₂S test as used as a presence/absence method to test the microbial quality of water, usually sodium thiosulfate is used as the precursor for hydrogen sulfide generation. In the conventional test, the sulfur-bearing amino acid L-cysteine is commonly added to improve detection of fecal indicator bacteria. However, experiments conducted with the methods and systems of the present invention have shown that L-cysteine and its dimer L-cystine are unstable and potential sources of false sulfide alarms. Experiments using the sensitive detection methods and systems of the present invention, have demonstrated that very stable, robust and reproduceable test results are achieved when L-taurine, i.e. an alkane sulfonic acid/ alkanesulfonate, is included in the CDM formulation. Excellent results were obtained when using L-taurine as a supplement to the CDM formulation.

If desired for the particular microbial test, using the method of this invention, thiosulfate may be further included in the CDM. However, tests to date to detect FIB have shown that thiosulfate can be omitted from the formulation if L-taurine is included. The inclusion of L-taurine or other alkanesulfonate compounds in the cultivation and detection medium as a more chemically stable alternative to L-cysteine improves the overall stability of the CDM, with the anticipated advantages of longer storage life and enhanced tolerance to higher temperatures, as typically used for sterilization.

The culture medium may be suitably adapted for use in the method and system.

For instance, molecules bearing sulfur in the form of a sulfonyl group show improved stability under the incubation conditions may be used.An alkane sulfonic acid or an alkanesulfonate is a compound of the formula R-(S=O)₂-OM, wherein M is H or a metal ion and R is an organic alkyl group - typically having 1 to 12 C-atoms, preferably having 2 to 6 C-atoms, more preferably having 2 or 3 C-atoms - optionally substituted with a suitable group such as with an amine group or a hydroxy group.

The dialkyl sulfone compound is a compound of the formula (alkyl)₂SO₂ wherein "alkyl" means an organic alkyl group - typically having 1 to 12 C-atoms, preferably having 2 to 6 C-atoms, more preferably having 2 or 3 C-atoms - optionally substituted with a suitable group such as with an amine group or a hydroxy group.

The culture and detection medium may include an alkane sulfonic acid, and such a particular alkane sulfonic acid is L-taurine.

The culture formulation suitably includes further typical ingredients known for the purpose of culturing. For instance, further included may be, without being limited to: soy peptone and/or yeast extract as sources of carbon; nitrogenous compounds; amino acids; minerals; and vitamins that are essential for growth. Further, bile salts are suitably included to favor the selective growth of *E.coli* and other enteric bacteria. Sodium chloride adds salinity and maintains osmotic balance. Suitable buffers are usually included, such as disodium hydrogen phosphate, to buffer the pH. Optionally, L-tryptophan may be included to enable the indole test. Oxalic acid may be included for added pH control and to scavenge any residual chlorine from disinfected water samples. H₂O is used to prepare the formulation as an aqueous medium. In this embodiment the CDM is prepared as a stock solution for addition to the test sample at the beginning of a test. The CDM should be sterile.

An example of a cultivation and detection medium (denoted "CDM-1") that is used for the detection and quantitation of fecal indicator bacteria is presented in Table 1.

**TABLE 1: Cultivation and detection medium CDM-1.**

| **No.** | **Ingredients** | **Amount** | **Unit** |
|---|---|---|---|
| 1 | Yeast extract | 2.1 | g |
| 2 | Soy peptone | 14.00 | g |
| 3 | NaCl | 3.30 | g |
| 4 | Na₂HPO₄ | 1.50 | g |
| 5 | Bile salts | 2.10 | g |
| 6 | L-tryptophan | 0.30 | g |
| 7 | L-taurine | 0.10 | g |
| 8 | Deionized H₂O | 74.0 | g |
| 9 | Oxalic acid, 1% (w/v) | 1.00 | ml |

The ingredients are added to a 100 ml wide neck LDPE screw cap bottle according to the sequence indicated. The bottle is shaken to dissolve the ingredients, then placed in a water bath at 90°C for 45 minutes to sterilize. This formulation is used to test water samples for fecal indicator bacteria at a cultivation temperature of 37-38°C.

Table 2 shows a formulation (denoted "CDM-2") that can be used to test water samples for thermotolerant coliforms at an incubation temperature of 44°C. A lower concentration of bile salts is used in this formulation in deference to the higher incubation temperature.

**TABLE 2: Cultivation and detection medium CDM-2.**

| **No.** | **Ingredients** | **Amount** | **Unit** |
|---|---|---|---|
| 1 | Yeast extract | 2.1 | g |
| 2 | Soy peptone | 14.00 | g |
| 3 | NaCl | 3.30 | g |
| 4 | Na₂HPO₄ | 1.50 | g |
| 5 | Bile salts | 0.70 | g |
| 6 | L-tryptophan | 0.30 | g |
| 7 | L-taurine | 0.10 | g |
| 8 | Deionized H₂O | 74.0 | g |
| 9 | Oxalic acid, 1% (w/v) | 1.00 | ml |

The cultivation and detection media as described above should be sterilized immediately after preparation, using any suitable sterilization technique. Experiments conducted with the CDM to date show that for use in the testing of water for FIB, sterilization can be achieved by standing the sealed, medium-containing vessel in a water bath at 90°C for 45 minutes. While the medium has not yet been tested for long-term stability, it can be stored at 10-25°C for several weeks without showing any signs of degradation. When a water source is tested in duplicate, mixing one sample with CDM prepared three weeks previously, and mixing the parallel sample with freshly prepared medium, no loss in the precision of detection signals due to reagent degradation is observed.

The CDM formulations disclosed here are illustrative of media that have been successfully used in validation testing. Variations and modifications are possible to further embody or optimize the composition of the media, for instance to enable shorter analysis times. Those skilled in the art will appreciate that the composition of the above-mentioned formulation can still be modified, or some or all of the component features thereof can be equivalently replaced; these modifications or replacements do not cause the essence of the corresponding technical solutions to deviate from the scope of the technical solutions of the embodiments of the present invention. For example, the medium could be provided in the form of a freeze dried (lyophilized) solid, or impregnated on an absorbent substrate; the quantities of the listed ingredients could be modified; the formulation could include nutrient sources derived from the enzymatic digestion of proteins, nutrient sources derived from the hydrolysis of proteins, or nutrients not derived by either of the aforementioned methods; it could include any inorganic or organic sulfur-bearing molecule or other substance that plays a role in the metabolic generation of sulfide, including but not limited to: sulfates, thiosulfates, sulfites, sulfides, polysulfides, thiols, sulfanes, sulfoxides, sulfur polymers, amino acids, amino sulfonic acids, synthetic, stabilized or modified forms of organosulfur molecules, such as cysteine hydrochloride, N-acetyl-cysteine, various forms of peptone, etc.; it could include pyruvate or other substances to assist the growth of injured microrganisms; it could include any suitable agent or agents to promote selective growth of the target organism, including but not limited to: bile salts, deoxycholic acid, desoxycholate, lauryl sulfate, crystal violet, methylene blue, brilliant green, antibiotics, including tetracycline, ampicillin, penicillin, cefsulodin, and novobiocin; it could include oxygen scavengers to favor anaerobic growth conditions; it could include oxidizing or other agents to favor aerobic growth conditions; it could include chromogenic or fluorogenic agents for confirmatory testing, or other reagents to enable biochemical testing of the cultivated medium; it could contain a molecule that when acted upon by the enzyme β-glucuronidase releases a sulfide, or a sulfur-bearing nutrient, facilitating sulfide detection to reveal the presence of *E.coli;* it could contain a molecule that when acted upon by the enzyme β-galactosidase releases a sulfide, or a sulfur-bearing nutrient, facilitating sulfide detection to reveal the presence of coliforms; it could contain reagents for the removal of substances in the test medium which might impair the analysis, for instance complex-forming reagents could be added to remove heavy metal contaminants, or thiosulfate to scavenge residual chlorine.

The following examples show how the above-described cultivation and detection formulations can be used to create calibration curves for the quantitation of fecal indicator bacteria and thermotolerant coliforms.

### Preparation of Serial Dilutions from an Enriched Population of FIB

Since the fecal indicator group of bacteria includes several species that are sulfide producers, and it is not possible to know in advance of a test which bacterial strain will be responsible for a positive H₂S test, a real sewage sample was used to demontrate the plausibility of the novel test method disclosed in this invention. A 50 ml sample of supernatent liquid from untreated sewage was collected. Within 60 minutes of sample collection, a 5 ml volume of the liquid was added to a 50 ml screw cap bottle containing 20 ml Luria-Bertani broth. The vessel was incubated at 37°C to produce an overnight culture in accordance with common laboratory procedure. The enriched innoculate was assumed to contain a population of 05E+10 cfu/ 100 ml "H₂S-FIB". Six 1/100 serial dilutions of the enriched innoculate were prepared by successively transferring 3 ml of each innoculated dilution into 297 ml of the next lower dilution. Sterilized mains tap (drinking) water was used as the diluent. The serial dilutions were labeled "05E+08", "05E+06", "05E+04", "05E+02", "05E+00", "05E-02"cfu/ 100 ml.

### Test for FIB, Incubation at 37°C

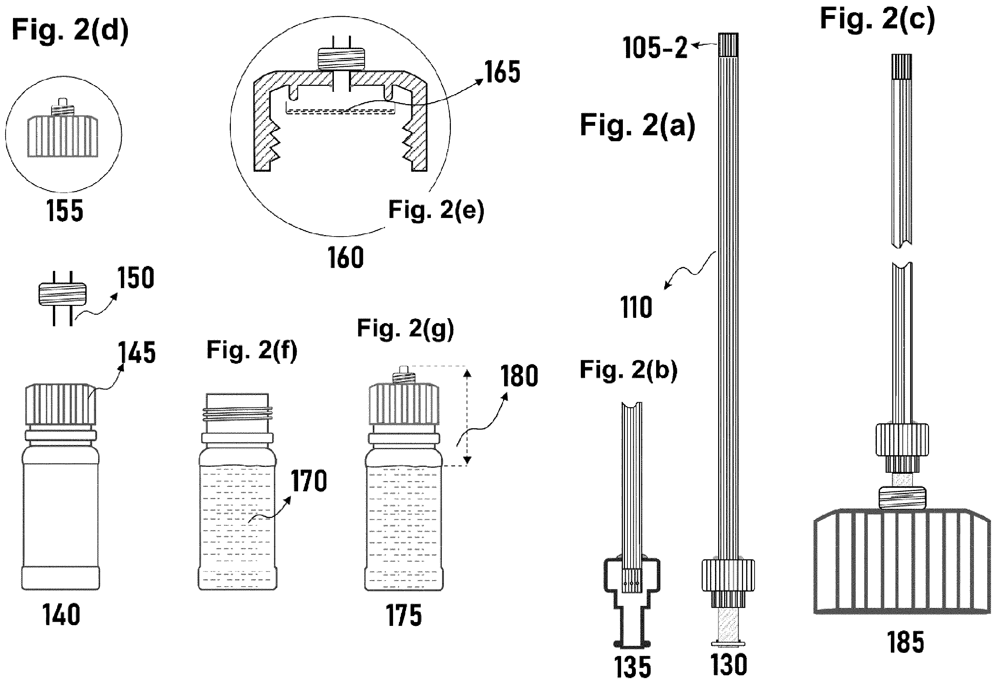

For each dilution two duplicate 50 ml water samples were tested in parallel. 50.0±0.1 ml of test sample was measured into each test bottle. 6.0 ml of the cultivation and detection medium "CDM-1" was added by pipette to each 50 ml sample and gently swirled to mix. The screw cap was fitted to the test bottle. A H₂S detector plug was attached to the cap (Refer to Fig. 2). All 12 test bottles were placed in a water bath incubator. The flat cable of each detector plug was attached to the measuring instrument. The temperature of the water bath was set to 37°C. The time (HH:MM) at which the incubation was started was entered into a spreadsheet accessible to the datalogger program. The datalogger was started. The time-to-detection for each H₂S detection event was computed by the spreadsheet, automatically transmitted by e-mail, and recorded.

### Test for Thermotolerant Coliforms, Incubation at 44°C

An enriched overnight innoculate and set of serial dilutions were prepared as described above. As previously, each innoculate dilution was to be tested using two duplicate 50 ml samples. 50.0±0.1 ml of test sample was measured into each test bottle. 6.0 ml of the cultivation and detection medium "CDM-2" was added by pipette to each 50 ml sample and gently swirled to mix. The screw cap was fitted to the test bottle. A H₂S detector plug was attached to the cap (Refer to Fig. 2). All 12 test bottles were placed in a water bath incubator. The flat cable of each detector plug was attached to the measuring instrument. The temperature of the water bath was set to 35°C for the first two hours, to aid recovery of any weakened organisms. The time (HH:MM) at which the incubation was started was entered into a spreadsheet accessible to the datalogger program. The datalogger was started. After the 2-h resuscitation period the incubation temperature was increased to 44°C. The time-to-detection for each H₂S detection event was computed by the spreadsheet, automatically transmitted by e-mail, and recorded.

Table 3 summarizes the data collected from both calibration tests.

**Table 3: Times-to-detection for serial dilutions of FIB, incubated at 37°C and 44°C (N.D.= no detection)**

| **Nominal Dilution [cfu/100 ml]** | 05E+08 | 05E+06 | 05E+04 | 05E+02 | 05E+00 | 05E-02 |
|---|---|---|---|---|---|---|
| **T_{d}, 37°C [hours]** | 2.90/3.01 | 4.91/4.99 | 7.19/7.21 | 9.29/9.50 | 10.91/- | N.D. |
| **T_{d}, 44°C [hours]** | 3.87/4.02 | 5.50/5.54 | 7.63/7.77 | 9.15/9.25 | 10.85/- | N.D. |

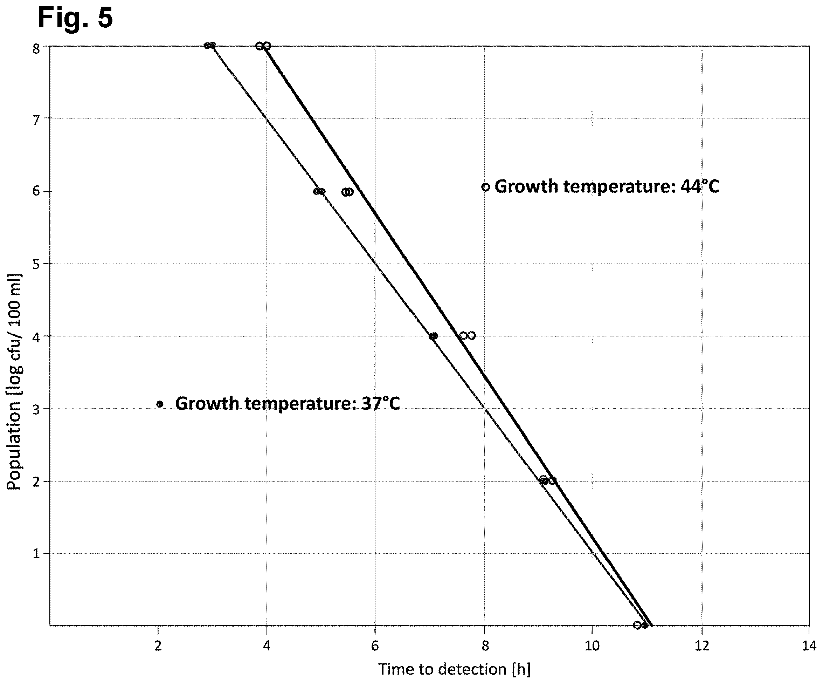

Fig. 5 shows calibration curves for the serial dilution tests at both 37°C and 44°C. The absence of detection signals from the 05E-02 dilutions suggests that no remaining organisms were present at this dilution level. This finding is consistent with the assumed starting population of 05E+10 cfu/100 ml in the enriched innoculate. The results show that the calibration curves for growth at 37°C and 44°C converge at the lower detection limit, corresponding to an incubation time of ca. 11 hours. The calibration curves demonstrate the linear relationship between the logarithm of the initial bacterial population and the time to sulfide detection. Enabled by the robust, sensitive detection method disclosed in this invention, it has been demonstrated that the bacterial population of a water sample can be quantitatively measured from the incubation time elapsed until a surge of sulfide is detected from within the test medium. This represents a major improvement over state-of-the-art H₂S tests, which are not capable of automated, quantitative measurement.

The incubation conditions disclosed here provide but two examples that have been successfully used in validation testing. No serious effort has yet been made to further optimize incubation conditions, for instance to enable shorter analysis times. Those skilled in the art will appreciate that the incubation conditions cited here can still be modified, or some or all of the component features thereof can be equivalently replaced; these modifications or replacements do not cause the essence of the corresponding technical solutions to deviate from the scope of the technical solutions of the embodiments of the present invention. This applies in particular to the incubation temperature, incubation times and the choice of aerobic, anaerobic or other growth conditions.

In another embodiment of the present invention the H₂S-detecting fuse is used to construct an apparatus suitable for the microbial testing of water and other media according to an automated most probable number method. A 100 ml or other volume of water is first partitioned into a plurality of wells or compartments. Each compartment is monitored by a H₂S-detecting fuse for the emergence of sulfide, according to the method already disclosed. At the end of a pre-determined incubation period, the H₂S-positive compartments are enumerated by a data-processing device. The bacterial population is computed from the number of H₂S-positive compartments and by reference to MPN tables. This embodiment of the invention, which enables an automated version of the MPN method, identifies a further improvement in the state of the art over MPN methods that require the attention of a skilled operator to visually inspect and complete an MPN test.

Any liquid or agar culture medium that is currently used for the selective detection, isolation or enumeration of organisms, based on the generation of sulfide as a metabolic product following a period of incubation, can be modified to allow its use in accordance with the automated method disclosed in this invention. Such modification usually involves the removal from the culture medium of the ferric ammonium citrate or other metal reagent added as a color indicator, so as to favor the release of H₂S for detection by a gas-detecting device. If desired, the H₂S-detecting fuse or an insubstantial modification of the embodiment disclosed here, may be immersed in an agar or other growth medium, rather than positioned in the headspace of the said medium. If required, other aspects such as the pH, choice of sulfide precursor, etc. are easily modified and optimized by experimentation. Several techniques commonly employed for the differentation of microorganisms, for instance the Sulfide-Indole-Motility (SIM) method, may beneficially combine the visual detection of color and other changes to the culture media with the automated detection of sulfide. Use of the H₂S-detection fuse enables remote sulfide detection from within a slant or similar test tube culture without blackening of the culture medium, and enables biochemical testing without possible interference of metal indicators in the culture medium. While it would overburden this disclosure to name all culture-based media that employ sulfide detection for the isolation or enumeration of bacteria, some harmful bacteria for which plating and related test methods are routinely used include *Clostridium perfringens, Salmonella spp.* and *Citrobacter freundii.* The method described in the present disclosure may also be applied to automated testing for the antibiotic resistance of sulfide-producing pathogens, using a culture and growth medium spiked with increasing concentrations of the antibiotic under study.

A concern raised by researchers with respect to the detection of H₂S as a method of testing water for the presence of FIB is that the test is likely to be sensitive to sulfide producing bacteria that are prevalent in the environment, though not of human or animal origin. While such concern is founded, it is also true that species of sulfide producing bacteria whose natural habitats are soil and aquatic environments, for instance the genus *Desulfovibrio,* tend to have slower growth rates than bacteria of enteric origin and may therefore fall outside the detection timeframe of the H₂S test. This is likely a contributing factor to the well-documented success of the presence/ absence H₂S test in detecting FIB and pathogens. In this respect, the shortened incubation times of the present invention, enabled by the highly sensitive, early detection of H₂S, represents a substantial improvement over the widely used P/A H₂S method. Other factors that are employed in the test method of the present invention to favor detection of enteric bacteria are the inclusion of bile salts (or other inhibitors such as crystal violet) and the incubation temperature (37°C or 44°C).

*Escherichia coli* is the organism favored by most regulatory authorities as an indicator of the fecal contamination of water. It may not be possible, using the method of this invention, to contrive a cultivation and detection medium capable of detecting all strains of *E.coli,* based on their generation of sulfide. Importantly, the direct detection of *E.coli* based on its production of H₂S is not necessary in order to detect its presence in a water sample. The high positive predictive value of the H₂S test for FIB, as confirmed by many scientific studies to date and enhanced by the method of the present invention, enables screening for FIB-positive water samples, allowing their early identification for further confirmatory testing.

Experiments conducted within the scope of this invention have demonstrated that the detection of hydrogen sulfide reveals the readiness of a cultivated water sample for the indole test. That is, a sample of the test water withdrawn from the culture vessel prior to the detection of H₂S will invariably show a negative reaction to Kovacs' reagent, while a sample tested subsequent to H₂S detection will invariably show a positive result. This relationship has been observed throughout the detection range from lowly- to highly polluted waters. It is a significant finding given the relatively short incubation timeframe (typically 12-13 hours) used by the present invention for detection of FIB. (The indole test is commonly conducted after 24 or 48 hours' incubation). Practice has shown that the indole test with Kovacs' reagent can be conducted from about 20 minutes onwards after H₂S detection. Several other microorganisms besides *Escherichia* show a positive reaction to the indole test. A positive test can therefore not be regarded as confirmatory for *E.coli.* However, a positive indole test does narrow the field of indole-positive strains considerably, as common sulfide producers such as *Citrobacter freundii, Campyllobacter, Pseudomonas aeruginosa, Salmonella spp.* are indole negative. A negative reaction to both the H₂S test and to Kovacs' reagent indicates with a high confidence level that the sample is free of *E.coli.* The indole test can therefore be applied as a confirmatory test to the H₂S test to guard against the risks associated with false negatives.

### Example of Confirmatory Indole Test

A H₂S test according to the method of this invention, as described above for the incubation of test samples at either 37°C or 44°C, is initiated. If FIB are present in a given test sample a H₂S detection signal will be issued by the test apparatus after a certain time during incubation. From about 20 minutes onwards, after the H₂S detection signal has been issued, the test can be checked for the formation of indole. A 3 ml volume of the test liquid is transferred from the test bottle into a test tube. 5 drops of Kovacs' reagent are added and the tube is swirled. The formation of a red ring on the liquid's surface indicates a positive result. Test vessels which have not issued a H₂S detection signal by the end of the testing period should also yield a negative result when tested, as described, with Kovacs' reagent. If a water sample incubated at 44°C to test for thermotolerant coliforms tests positive for both H₂S and indole, the sample is very likely positive for *E.coli.*

Experiments conducted within the scope of this invention have also demonstrated that the H₂S test disclosed here can be combined with a confirmatory test based on enzyme-specific chromogenic/ fluorogenic media. Aliquots withdrawn from a positive H₂S test are added to the enzyme-specific medium and cultivated for a further 2 hours to allow identification of fluorescing samples. This cultivation period has been chosen conservatively to allow more certainty in visually identifying fluorescence, and could likely be shortened considerably. Water samples that fail to respond to the H₂S test invariably also show a negative response to the enzyme-specific test for *E.coli.*

### Examples of Confirmatory Enzyme-Specific Tests

Experiments demonstrate that a positive or negative result obtained by the test method of the present invention may also be validated by confirmatory testing against enzyme-specific reagents. For this purpose the market-leading IDEXX chromogenic/ fluorogenic test kit Colilert-18^{®} was employed. Total coliforms present in the test sample act upon the Colilert-18^{®} chromogenic substrate to produce a yellow color following incubation. *Escherichia coli,* if present, act upon a fluorogenic substrate to release a fluorescent substance after a suitable incubation period. In experiments conducted within the course of this invention, the Colilert-18^{®} test was run both in parallel and in series with the novel H₂S test.

For the purpose of the experiment, 1 liter of sterilized tap water, spiked to a nominal FIB population of approximately 05E+02 cfu/ 100 ml was prepared by the serial dilution technique described above. Colilert-18^{®} reagent was added to six sterile 100 ml non-fluorescent test bottles, in accordance with the manufacturer's instructions. Three of the bottles were filled with 100 ml of the polluted water sample. A further three blank samples were prepared by the addition of 100 ml sterilized, innoculate-free tap water to the remaining three test bottles. Three 100 ml test samples of the innoculated water, plus three blank samples containing 100 ml of sterilized water, were prepared in accordance with procedures already described for the H₂S test of the present invention. The six test bottles with the added Colilert-18^{®} reagent were incubated at 35°C in accordance with the manufacturer's instructions. The six samples with added "CDM-1" reagent as used for the H₂S test were each fitted with a H₂S detector plug, connected to the test apparatus and incubated at 37°C. After ca. 8 hours of incubation the FIB-innoculated bottles charged with the enzyme-specific reagent had begun to develop a yellow color, indicating the presence of coliforms. At this stage these bottles did not display fluorescence when irradiated by a UV lamp. After approximately 8.5 hours of incubation, the innoculated samples containing enzyme-specific reagent showed weak fluorescence. After 8.9 hours, the three samples innoculated for the H₂S test registered H₂S detection. At this time, the three innoculated Colilert-18^{®} test samples showed intense fluorescence, confirming the presence of *E.coli.* The three blank test samples with added Colilert-18^{®} reagent showed no development of yellow color, and were also negative to the fluorescence test when examined in accordance with the manufacturer's instructions 18 hours after the beginning of incubation.

The Colilert-18^{®} test kit was also used to post-check the results of the H₂S test method, following a waiting period of about 20 minutes from the time of H₂S detection. 1 ml of liquid was withdrawn from each of the three H₂S-positive test vessels and added to bottles containing 15 ml of Colilert-18^{®} reagent, prepared from sterilized water in accordance with manufacturer's instructions. In a similar manner, 1 ml of test liquid was transferred from each of the three H₂S-negative test vessels to bottles containing 15 ml of the enzyme-specific reagent. The six test bottles were incubated at 35°C. After approximately 60 minutes, the three H₂S-positive samples had developed a yellow color, confirming the presence of total coliforms. Following a further incubation period of about 60 minutes, the H₂S-positive samples all tested positive for fluorescence, confirming the presence of *E.coli.* At the end of the 2-hour incubation period, the H₂S-negative samples had neither developed a yellow color, nor were fluorescent when viewed under UV light. The three blank samples were still negative to the enzyme-specific tests when examined in accordance with manufacturer's instructions 18 hours after the start of incubation. Similar experiments conducted with lower and higher bacterial contamination levels yielded similar test outcomes. These experiments demonstrate that a positive H₂S test result can be verified by a simple check against an officially-approved enzyme-specific test method. Moreover, the H₂S test method of the present invention can be run parallel to an approved enzyme-based test method to remotely inform the analyst of test samples with high levels of *E.coli,* well in advance of the incubation period prescribed for the enzyme-specific test method.

The sealable vessels used in the test method of this invention are standard wide-neck HDPE chemical bottles with screw cap. For the apparatus constructed to demonstrate this invention, a hole is bored in the screw cap to allow a male luer fitting to be mounted and sealed on the outside of the cap. The end of the FFC cable with electrodes and disposed chemiresistive film is enclosed within a separate female luer fitting. When the assembled female luer fitting (the "H₂S detector plug", refer to Fig. 2) is connected to the male fitting on the screw cap, the detecting film has direct access to the vapor space of the culture medium contained within the test bottle. This design allows a single-size H₂S detector plug to be attached via the same luer connector to test vessels of varying test volumes. The non-detecting end of the FFC cable is attached via a snap-in connector to the test instrument.

Although the use of a moisture barrier between the liquid test medium and detector fuse is not strictly necessary for detection of the sulfide, a moisture barrier improves the sharpness of the detection signal, and is also desirable to isolate the single-use detector plug from a potentially enriched bacterial culture. For this purpose a hydrophobic microporous cloth filter is used. To construct the filter used to demonstrate this invention, three circular pieces (ϕ30mm) of microfilter fabric, 0.2µm, HEPA H14, are impregnated with a hydrophobic spray and dried. They are then assembled into a 3-layer filter that is positioned within the screw cap of the test vessel, as shown in Fig 2.

In summary, the present invention offers many advantages over state-of-the art techniques for the detection and enumeration of bacteria. The incubation vessel is re-useable, leaving only a miniature detector plug and the cultivated test medium as inexpensive consumable items. Test sample size can be chosen to suit the individual analysis without the need for different detector fittings. To start a test, the medium is simply mixed with the CDM and incubated, without prior sample preparation, irrespective of the anticipated bacterial population. If required to expedite the test result, pre-concentration of samples with anticipated low bacterial counts is possible, using membrane filtration. The sample can be incubated at 37°C to test for FIB, or at 44°C to test for thermotolerant coliforms. If further confirmatory tests are desired or prescribed, they can be initiated at an early stage, following notification of a positive H₂S test, without the need to await the end of a lengthy incubation period. The analyst is notified electronically of the test result. In low-resource countries, this last feature would allow a technician to commence several tests at remote locations within the same day, since the test results could be received remotely by smartphone. The automated, signaling test method according to the present invention could be conducted in a small, dedicated work area, without the need for sophisticated laboratory equipment.

### Brief Description of Drawings

This section provides an overview of the drawings appended to this application. Further commentary on each drawing is provided below. Note that the drawings are intended as schematics and are not drawn to scale.
Figs. 1(a) to (c) shows an embodiment which illustrates how a flat flexible cable (FFC) is used as a simple base and means of constructing the gas-detecting device (sometimes also called "gas-detecting fuse") of this invention.
Figs. 2(a)-(g) shows embodiments which illustrate a sealable vessel, that contains a test sample or test medium, to which a gas-detecting device is mounted.
Figs. 3(a) and (b) respectively illustrate sealed bacterial cultivation vessels, showing two examples of how a gas-detecting device could be mounted thereon.
Fig. 4 illustrates how the gas-detecting device (gas-detecting fuse) of the present invention is part of, connected to, or attached to a measuring instrument which includes electronic evaluation and signaling devices, according to an embodiment of the present invention.
Fig. 5 illustrates calibration curves that relate the initial bacterial population of a test medium to the cultivation time elapsed until sulfide detection, respectively representing calibration curves for incubation at 37 (for FIB) and 44C (for thermotolerant coliforms).
Fig. 6 shows the datalog of a typical microbial water test obtained when performing an embodiment of the method of the present invention.
Fig. 7 shows the datalog of the test described in Example 2 of the present disclosure.
Fig. 8 provides an overview of tests conducted on selected water sources using the method of the invention.

### Detailed Description of Drawings

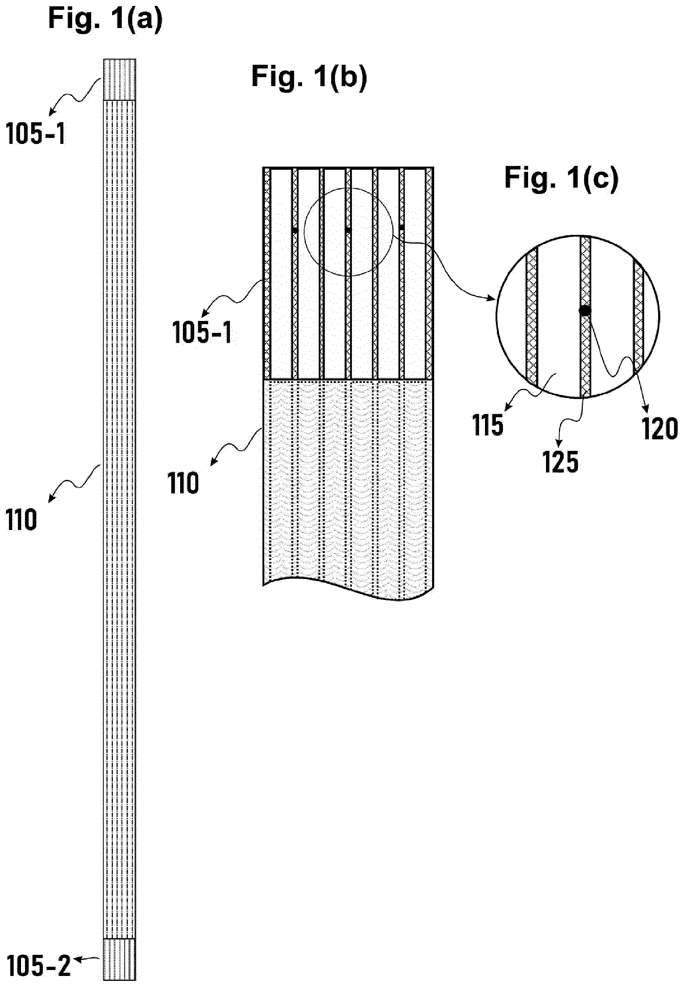

With reference to Figs. 1(a) to (c), an FFC cable ("A" type, with same-side connectors) is shown. The non-insulated sections 105-1 and 105-2 at the ends of the cable each have six exposed tin-plated copper conductors that are commonly referred to as "pins". The rest of the cable 110 is insulated with a polymer coating. For the purpose of this invention the conductors are used as a set of electrodes to measure the change in resistance of an applied, gas-sensitive film. The measurement of resistance or impedance can be achieved in numerous ways. Also, the measurement of an irreversible change in the electrical or magnetic properties of a material when exposed to a reactive gas such as hydrogen sulfide can also be achieved in numerous ways, other than by measuring a change in resistance as shown and described in this particular embodiment. For example, as an alternative to the use of a chemiresistive film in the present embodiment, a dielectric material or ferrimagnetic film is used for the detection of a gas by monitoring changes to capacitance or inductance respectively. Accordingly, the resistance measurement technique described here should not be interpreted as a necessary embodiment, or as otherwise defining or limiting the scope of the invention.

The FFC cable as depicted has a pitch of 0.5mm, which is the distance between the center of a conductor 115 and the center of a nearest-neighboring conductor. According to the example already described, the chemiresistive film is prepared as a paste and applied as a spot 120 on to the insulating substrate 125 so as to contact two nearest-neighbouring pins. In the embodiment depicted in Fig. 1(b), three spots of paste are applied to three pairs of electrodes. Given the high sensitivity to the target gas and otherwise intrinsically advantageous properties of the chemiresistive film, the application of a plurality of spots is not necessary for the correct functioning of the device. As depicted here, multiple spots are applied to increase reliability by rendering each individual spot redundant. After drying, the resistance of each spotted film typically lies in a range of about 1 MΩ to 1000 MΩ. As depicted in Figs. 1(a) and (b), the upper connector 105-1 of the FFC cable serves to create the gas-sensitive film. The lower connector 105-2 serves to electrically connect the chemiresistive film to a measuring device. In the example shown in Fig. 1, the pins of 105-2 are connected to the measuring device as three parallel resistors. While the assembly depicted in Fig. 1 provides an example of how a gas-detecting fuse can be embodied by simple means, a market-ready device would be more suitably fabricated by ink jet printing or other mass-production technology, for instance in the form of an adhesive tag.

Figs. 2(a)-(c) illustrate how the gas-detecting end 105-1 of the FFC cable is encapsulated within a plastic luer lock cap with female connector to form a gas detector plug 130. Reference sign 135 shows a cut-away view of the same gas detector plug, depicting how the gas-detecting device (gas-detecting fuse) is positioned within the plug. 2-component epoxy glue is used to seal a slit at the top of the plug through which gas-detecting end 105-1 of the FFC cable is inserted.

In the present embodiment depicted as Fig. 2(d), the examples of water testing described in this disclosure were conducted using a wide-necked HDPE bottle 140 with screw cap 145. A Ø6mm hole was bored in the screw cap, allowing a plastic luer fitting with male connector 150 to be mounted on it. The screw cap with luer connector, 155, was sealed with 2-component epoxy glue. Reference sign 160 shown in Fig. 2(e) provides a cut-away view of the screw cap assembly, showing a filter 165 that is positioned within the screw cap to isolate the gas detector plug 130 from the bacterial culture, while allowing gases to access the gas-detecting fuse. The filter is hydrophobic in order to reduce the amount of condensed moisture on the chemiresistive film, thereby improving gas contact and gas detection. Any other suitable filter insert, such as a porous plastic frit, impregnated paper disc or filter butt, can equivalently be used for this purpose.

To construct the filter used to demonstrate this embodiment, three circular pieces (ϕ30mm) of microfilter fabric, 0.2µm, HEPA H14, are impregnated with a hydrophobic spray and dried.

They are then assembled into a 3-layer filter that is positioned within the screw cap of the test vessel, as shown by reference sign 165. The cone seal lip within the screw cap serves to hold the filter inlay in place. As shown in Figs. 2(f) and (g), to test a water sample for the presence of microorganisms, suitable volumes of water sample and cultivation and detection medium (CDM) 170 are transferred to a sterile test vessel. In the examples described in this disclosure, 6 ml CDM were added to 50 ml water sample and mixed.

The test volume of water and amount of added CDM cited in this disclosure are provided solely for reasons of illustration. Any suitable amount of test volume and a suitable ratio of added CDM can be used, or the composition of the CDM can be optimized, for instance to expedite the time to bacterial sulfide production and detection. Accordingly, the details here for the test method of the invention are merely illustrative and shall not in any way limit its scope. Reference sign 175 depicts the test bottle used in this example with its screw cap fitted. The volume above the liquid surface within the test bottle, as shown in this example, is occupied by gases and vapors and is generally referred to as the "headspace" 180. Fig. 2(c) and reference sign 185 shows the gas detector plug 130 being attached via its female luer connector to the male luer connector of the screw cap. Assembled as described, the gas-detecting fuse is in direct contact with the headspace of the test bottle.

While the example depicted in Figs. 2(a)-(g) employs a laboratory bottle as the cultivation vessel, any suitable vessel could be conceived and designed for this purpose. As one advantageous design mentioned here by way of illustration, a syringe serves as the test vessel. In this manner the water sample is taken directly from a river or pond without the use of a graduated cylinder, pipette, etc to measure the water sample into the test vessel. Prior to sucking the water sample into the syringe, the CDM is added to the syringe as a pellet or powder from a sterile sachet. By way of example, graduation marks on the syringe are used to collect a 50 ml water sample. With the syringe held in an upright position, 10 ml of air is drawn to create a gaseous headspace. The syringe is placed in a suitable rack, a detector plug like the one shown by 130 in Fig. 2(a) is attached to the male luer connector of the syringe, and the assembly is incubated as described in the present disclosure. The ability to directly test a liquid sample of desired volume using a syringe connected to a gas detector plug, via a single-size fitting, illustrates a further advantage of the present invention over other state-of-the-art methods.

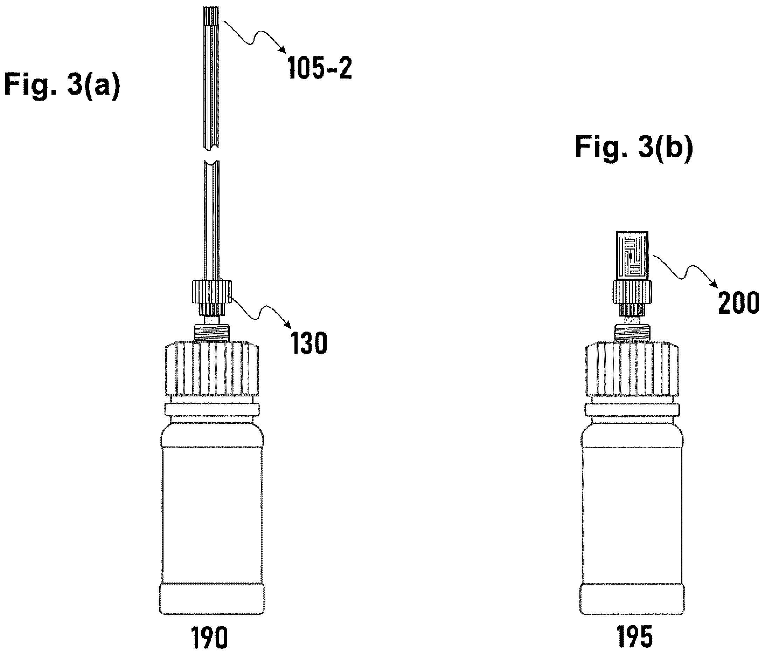

Figs. 3(a) and (b) illustrate two embodiments respectively using individually adapted or modified test vessels with connected gas detector plugs. In Fig. 3(a), 190 depicts a test bottle with mounted gas detector plug 130, as described for the embodiment shown in Fig. 2. In Fig. 3(b), 195 illustrates a test vessel according to another embodiment of the present invention which allows wireless transmission of a sulfide detection event to a remote instrument or reader. The drawing depicts an RFID tag 200 mounted upon the screw cap of a test vessel 195 in such a way that a suitable gas-sensitive film is in contact with the gas/ vapor headspace inside the test vessel. The gas-sensitive film corresponds to the gas-detecting fuse disclosed in the present disclosure. In such an embodiment the gas-detecting fuse is electrically connected via the screw cap to a suitable section of the RFID tag's IC. A decrease in the ohmic resistance of the gas-detecting fuse is used to switch on or off an electronic circuit, thereby modifying the signal transmitted from the RFID tag to an RFID reading device.

As an alternative embodiment, the gas-detecting fuse uses a ferrimagnetic film disposed upon the antenna of the RFID tag. The RFID tag is fabricated as an adhesive label, to be adhered over an orifice in the screw cap in such a way that the ferrimagnetic film will have contact to the gases within the headspace of the test vessel. When acted upon by the sulfide, the inductance of the ferrimagnetic film is altered, thereby causing a shift in the frequency of the signal transmitted between RFID tag and its reader.

By way of the embodiments of Figs. 3(a) and (b) as well as the above described modifications, the gas-detecting fuse of the present invention can easily be implemented to enable remote monitoring of incubated test vessels. Moreover, such an embodiment allows a large number of test samples, each with its own remotely identifiable sample number, to be monitored with one single reader instrument, while obviating the need to manually connect the test vessels to a measuring instrument. The ability to incubate a test sample from a remotely positioned test instrument is facilitated by the simplicity of the gas-detecting fuse of the present invention. It would be considerably more difficult to remotely monitor an incubating sample using conventional testing methods that depend on optical connections, immersed electrodes, etc.

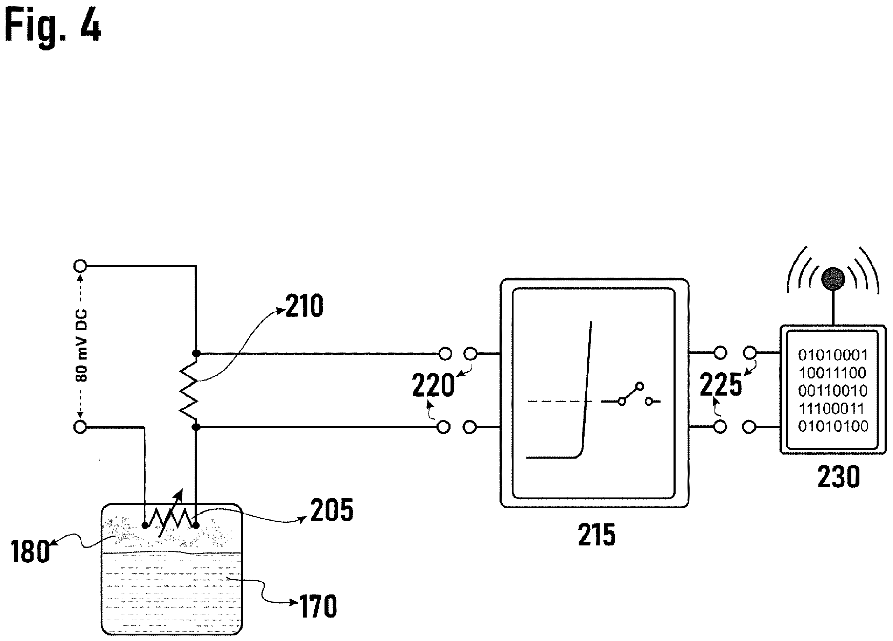

Fig. 4 shows an embodiment of a measuring instrument comprising an electronic evaluation and signaling assembly, and schematically illustrates an example of how the gas-detecting device (gas-detecting fuse) may be part of, or connected with, or attached to such a measuring instrument. In this embodiment, a gas detecting device (gas-detecting fuse) 205 is depicted as a variable resistor positioned within the headspace 180 above a liquid medium under cultivation, 170. In the apparatus used to demonstrate the present invention, a test voltage of 80 mV is applied across a circuit connecting the gas-detecting fuse and an in-series reference resistor 210. The reference resistor is depicted as being located outside of the test instrument for reason of schematic clarity. The test voltage and value of the reference resistor cited here were arbitrarily chosen. Those skilled in the art will appreciate that higher or lower test voltage values could readily be applied to monitor the resistance of the gas-detecting fuse. The use of a lower test voltage, in conjunction with signal amplification circuitry, would advantageously prolong battery life if the test method of this invention were to be embodied as an apparatus intended for mobile use. In the apparatus used in an illustrative example of the present invention, the measuring electronics were powered by 5V DC from the USB connector of a laptop on which a datalogging software was run. The schematic shows how the reference resistor 210 is connected to the measuring instrument 215 via terminal connectors 220.

As described for Fig. 3, the increase or decrease in the value of an electrical or magnetic property upon contact with sulfide is communicated from the test vessel to the measuring device by means of wireless transmission.

The signal-measuring device 215 monitors the voltage across the reference resistor and issues a warning signal when a pre-determined voltage threshold is crossed. The warning signal is sent to a computing and signaling device 230 that calculates the time elapsed from the start of incubation until sulfide detection. The device computes a bacterial population based on the time-to-detection of sulfide and transmits the result as a short message via e-mail or other electronic means to a user or analyst or any interested party.

In one embodiment of the present invention, the signal-measuring device is a dongle, a USB stick or a similar apparatus that is attached to a computer. In said embodiment, the signal-measuring device notifies the computer that a voltage threshold had been crossed. Based on this input, the computer calculates the elapsed time to detection, computes a bacterial population from an electronically-stored calibration curve of bacterial population Vs time-to-detection of sulfide, composes a short e-mail or other electronic message that contains the test result, and transmits the result via e-mail or by other electronic means to the user or analyst or any other interested party.

In another embodiment the measuring and signaling functions are combined within a single instrument that monitors the gas-detecting fuse either via wired connectors or via wireless connection. Such an instrument incorporates a user interface to allow input of key information such as sample ID, test incubation temperature, etc. It may also incorporate a START button that automatically records the starting time of incubation when pressed, or an equivalent touchscreen function.

Fig. 5 shows two calibration curves, one each for media incubated at temperatures of 37°C and 44°C, which were prepared from test data as described in the present disclosure. The graph provides a calibration curve of log N₀ Vs t_{d}, where N₀ is the number of microorganisms present at the beginning of the incubation period and t_{d} is the time elapsed until sulfide detection. Surprisingly, the detection of a surge in the quantity of sulfide produced by a bacterial population by the method and system of the present invention shows a precisely linear relationship between log Nₒ and t_{d}, achieving a correlated linear relationship which, to the best knowledge of the inventor, has not yet been measured with this precision in the prior art. Therefore, results and linear calibration curves produced by the method and system of the present invention enable the precise automated computation of the level of sulfide-producing micro-organisms present in a test medium, based on the incubation time elapsed until a surge of sulfide is detected. Whereas the manual H₂S test for fecal indicator bacteria relies upon human observation to detect the gradual blackening of a cultivated water sample, the present invention demonstrates that the generation of sulfide by a bacterial culture occurs abruptly, following an elapsed incubation period that is a characteristic of the bacterial strain, and is evidenced by a sharp detection signal. The ability of the method disclosed in the present invention to detect the production of sulfide from media cultivated at 44-45°C is also significant, since it is generally recognized that the 44.5°C incubation temperature favors the growth of enteric bacteria over general coliforms. This aspect enables the quantitative measurement of FIB in a water sample cultivated at 44-45°C. As already disclosed, test samples showing a positive result for thermotolerant coliforms can be subjected to further biochemical test methods such as the indole test or enzyme-specific tests to confirm the presence of *Escherichia coli.* The method of the present invention thereby facilitates the routine screening of a large number of water samples for FIB while enabling quick confirmatory testing of samples showing positive results for the H₂S test.

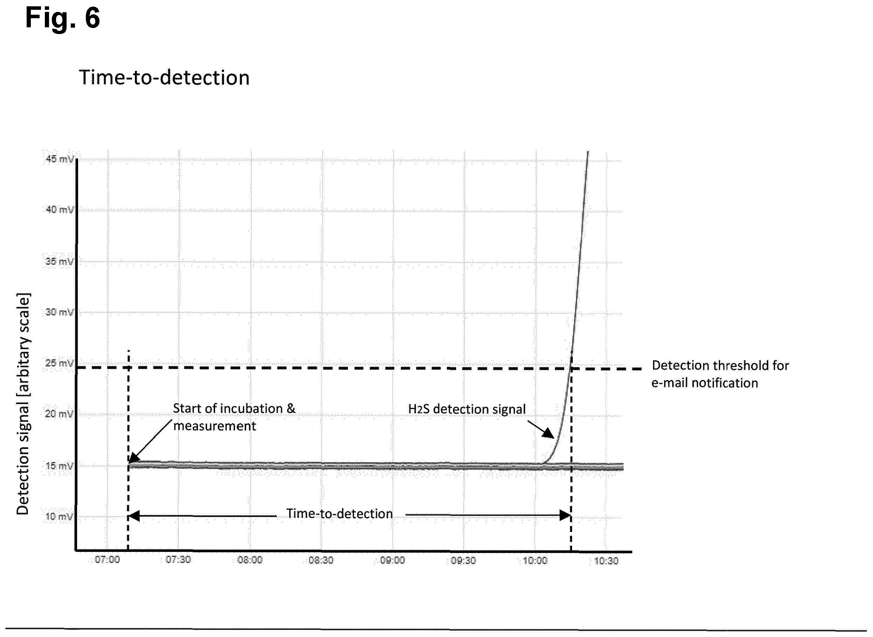

Fig. 6 shows the datalog of a typical test according to the method of the present invention. It is provided to illustrate some concepts described in this disclosure, such as "time-to-detection", "detection threshold voltage", etc. In particular, the "time-to-detection" value represents a period of time from the start of incubation of a test culture up to the time when a certain (a desired or prescribed or characteristic) threshold value of a measured parameter (here voltage as the indicator for resistance/film resistivity) has been reached.

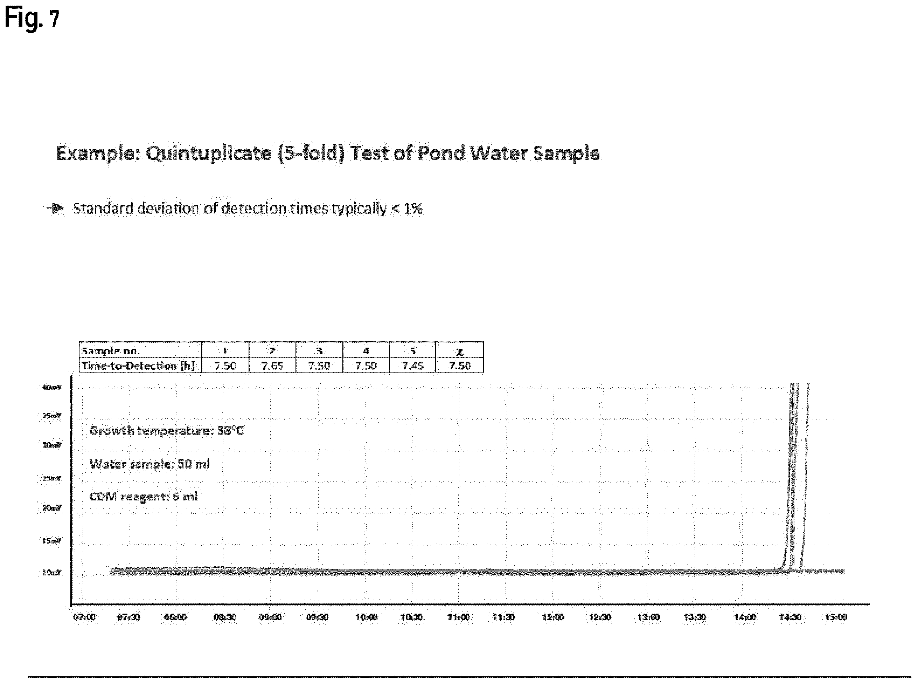

Fig. 7 illustrates the datalog of a test culture incubated at 38°C, as described under Example 2 of this disclosure. This typical test result is provided to illustrate the high degree of precision enabled by the test method of the present invention. As illustrated by the datalog, and facilitated by the chemically-stable CDM of the present invention, the measurement signal yields a very stable baseline, corresponding to a high ohmic resistance of the gas-detecting fuse, until the abrupt emergence of sulfide after an incubation period of approximately 7.5 hours. The five parallel-tested water samples of this example return sharp detection signals within a time window of 12 minutes, following an incubation period of approximately 450 minutes. The median time-to-detection (in this example 7.5 hours) may be compared with an applicable calibration curve to compute the bacterial population of the tested water sample.

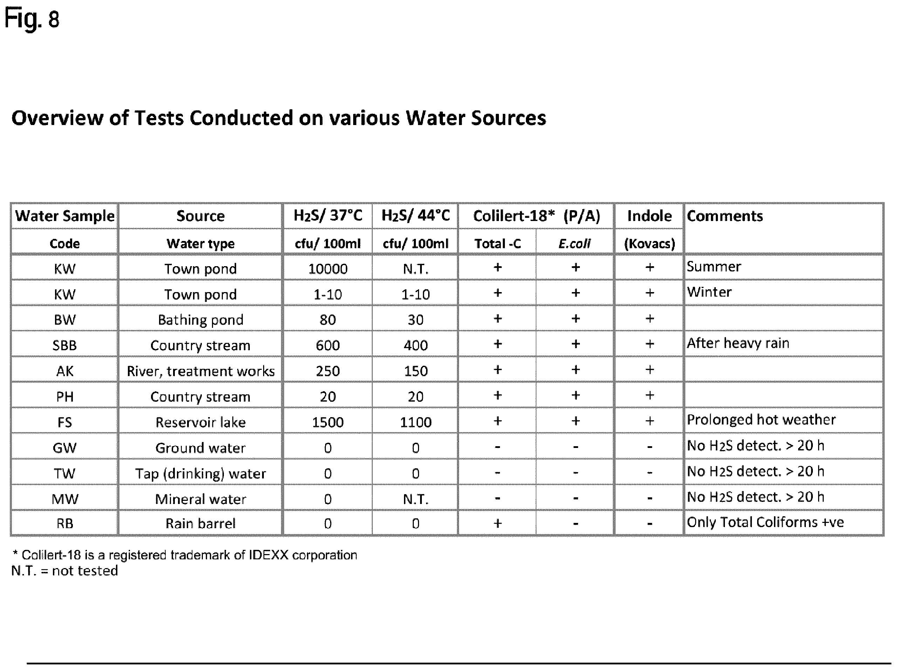

Fig. 8 provides a tabular overview of tests conducted on various samples of surface and drinking water, using the method and apparatus disclosed in this application. These results are provided to illustrate the feasibility, robustness and reliability of the automated H₂S test, based on tests conducted on real water samples. Results of indole tests and tests using the Colilert-18^{®} test kit are provided for comparison. Drinking and ground water samples were measured over a period of 20 h to demonstrate the absence of H₂S signals from FIB well beyond the normal duration of the test. The results show excellent correlation between the data obtained from the H₂S test compared with those of the indole and Colilert^{®} tests.

As already described, the test method of this invention can equally be applied to the detection of sulfide-producing microorganisms on many other applications, including laboratory cultivation tests, the testing of industrial installations for the presence of sulfate reducing bacteria, testing of clinical samples, etc. The selected results disclosed here for microbial water testing should therefore not be interpreted as defining the scope of the invention or as limiting its application in practice.

### Examples

### Example 1: Application of the invention to the testing of ground water.

### Test for FIB, Incubation at 37°C

A sample of about 1 liter of town ground water was collected from a hydrant. Testing of the ground water was conducted in triplicate. For test validation purposes, two 50 ml samples of water from a nearby pond were tested in parallel. 50.0±0.1 ml of test sample was measured into each of the five 50 ml test bottles. 6.0 ml of the cultivation and detection medium "CDM-1" was added by pipette to each 50 ml sample and gently swirled to mix. The screw cap was fitted to each test bottle and a H₂S detector plug was attached to the cap (Refer to Fig. 2). The five test bottles were placed in a water bath incubator. The flat cable of each detector plug was attached to the measuring instrument. The temperature of the water bath was set to 37°C. The time (HH:MM) at which the incubation was started was entered into a spreadsheet accessible to the datalogger program. The datalogger was started. The time-to-detection for each H₂S detection event was automatically transmitted by e-mail and recorded.

### Test for Thermotolerant Coliforms, Incubation at 44°C

A sample of about 1 liter of town ground water was collected from a hydrant. Testing of the ground water was conducted in triplicate. For test validation purposes, two 50 ml samples of water from a nearby pond were tested in parallel. 50.0±0.1 ml of test sample was measured into each of the five 50 ml test bottles. 6.0 ml of the cultivation and detection medium "CDM-2" was added by pipette to each 50 ml sample and gently swirled to mix. The temperature of the water bath was set to 35°C for the first two hours, to aid recovery of any weakened organisms. The time (HH:MM) at which the incubation was started was entered into a spreadsheet accessible to the datalogger program. The datalogger was started. After the 2-h resuscitation period the temperature was increased to 44°C. The time-to-detection for each H₂S detection event was automatically transmitted by e-mail and recorded.

The samples taken from the pond returned mean detection times of 9.1 hours for the samples incubated at 37°C and 9.6 hours for those incubated at 44°C. All six samples that were collected from the ground water failed to show any response over an incubation time of 20 hours. Indole tests conducted on the ground water samples at the end of the 20-hour period returned negative results for those tested at both the 37°C and 44°C growth temperatures. Indole tests conducted on the validation samples taken from the pond all yielded positive results.

### Example 2: Application of the invention to the testing of a polluted water source.

### Test for FIB, Incubation at 38°C

A sample of about 1 liter of water was collected from a stagnant town pond. Testing of the surface water was conducted in quintuplicate. 50.0±0.1 ml of test sample was measured into each of the five 50 ml test bottles. 6.0 ml of the cultivation and detection medium "CDM-1" was added by pipette to each 50 ml sample and gently swirled to mix. The screw cap was fitted to each test bottle and a H₂S- detector plug was attached to the cap (Refer to Fig. 2). The five test bottles were placed in a water bath incubator. The flat cable of each detector plug was attached to the measuring instrument. The temperature of the water bath was set to 38°C. The time (HH:MM) at which the incubation was started was entered into a spreadsheet accessible to the datalogger program. The datalogger was started. The time-to-detection for each H₂S detection event was automatically transmitted by e-mail and recorded.

The pond samples returned a mean time-to-detection of 7.5 hours, corresponding to a nominal bacterial population of 3200 cfu/ 100 ml. Indole tests conducted on 3 ml samples of liquid extracted from all five test cultures, about 20 minutes after H₂S detection, yielded positive results.

## Claims

1. A method for detecting the presence or quantitating the population of a microorganism in a sample, the method comprising:
(a) mixing the sample or part of the sample suspected to contain a microorganism with a culture medium (170), and applying culturing conditions which allow the generation of at least one volatile metabolic product by a microorganism, wherein the culture medium (170) in step (a) contains at least one sulfur-bearing molecule as a precursor to allow the generation of volatile sulfide by the microorganism of interest;
(b) detecting sulfide as a metabolic product, if generated, by a change, which said sulfide metabolic product induces in an electrical property or a magnetic property and/or an electromagnetic property of a gas-detecting device (130, 135), while the gas-detecting device (130, 135) is in contact with the vapor/gas phase above or adjacent to or in communication with the culture medium (170), and
(c) obtaining the result of a change in the electrical property or the magnetic property and/or the electromagnetic property of the gas-detecting device (130, 135) as an indication of the absence or the presence or the population of the microorganism in the culture medium (170), or respectively, in the sample,
wherein the duration of microbial growth, from the beginning of incubation until the emergence and detection of the sulfide metabolic product, is measured and used to compute the absence or presence or population of the microorganism in the test medium (170).

2. The method of claim 1, wherein in step (c) the change in the electrical or the magnetic property and/or the electromagnetic property generates a signal when a threshold value is crossed.

3. The method of claim 1 or 2, wherein the detection in step (b) involves partial or complete conversion into a metal sulfide or other electrically conducting sulfide.

4. The method of any one of claims 1 to 3, wherein step (b) involves use of a chemiresistive film whose electrical resistance is changed when contacted by gaseous or vapor phase sulfide.

5. The method of any one of claims 1 to 4, wherein obtaining the result in step (c) involves transfer of a signal, which was generated as a result of the change in the electrical or magnetic and/or electromagnetic property, to a remote instrument or to a remote user.

6. A system for detecting the presence or quantitating the population of a microorganism in a sample, comprising:
a culture medium (170) which allows the generation of at least one sulfide metabolic product by a microorganism;
a gas-detecting device (130, 135) arranged to detect at least one metabolic product generated by the microorganism by inducing a change of a property of the gas-detecting device (130, 135), the changed property being selected from the group consisting of an electrical property, a magnetic property, and an electromagnetic property; and
a measuring instrument arranged to detect a change in the electrical or the magnetic and/or the electromagnetic property of said gas-detecting device (130, 135),
wherein the gas-detecting device (130, 135) is arranged to detect organic or inorganic sulfide in gaseous or vapor form, wherein a metabolic product detected by the gas-detecting device (130, 135) belongs to the chemical family of sulfides,
wherein the gas-detecting device (130, 135) is in contact with the vapor/gas phase above or adjacent to or in communication with the culture medium (170) and generates a signal when a threshold value is crossed, thereby indicating the presence or the population of the microorganism, and
wherein the system is configured such that the duration of microbial growth, from the beginning of incubation until the emergence and detection of the sulfide metabolic product, is measured and used to compute the absence or presence or population of the microorganism in the test medium (170).

7. The system of claim 6, further comprising a signal processing and data transmission assembly that uses signals received from the measuring instrument to determine the presence or the population of the microorganism derived from the sample or the part of the sample, and transmit the result via electronic means to a remote user.

8. The method of any one of claims 1 to 5, or the system of any one of claims 6 to 7, wherein the metabolic product to be detected is allowed, during the course of microbial growth, to accumulate in any headspace (180) and/or other volume which adjoins or is in communication with the test culture, preferably wherein the headspace (180) or other volume is above or in communication with the medium (170) used for the cultivation of microorganisms within a sealed vessel.

9. The method of any one of claims 1 to 5, or the system of any one of claims 6 to 8, wherein the detection of sulfide signals that the test culture has reached a state of readiness for confirmatory biochemical or microbiological testing.

10. The method of any one of claims 1 to 5, or the system of any one of claims 6 to 9, wherein the active detecting material used for the detection of sulfide has a solubility product constant (Ksp) value which is less than 1x10⁻¹ at 25°C, and/or wherein the active component of the sensor is copper(1) oxide.

11. The method of any one of claims 1 to 5, or the system of any one of claims 6 to 10, wherein the response of the gas-detecting device to sulfide changes the electrical or magnetic or electromagnetic properties of a wireless signaling component, or activates a wireless signaling device (200, 215, 230), thereby announcing the detection or nondetection of sulfide to a reader, receiver, or other electronic device by wireless transmission.

## Patentansprüche

1. Verfahren zum Nachweis des Vorhandenseins oder zur Quantifizierung der Population eines Mikroorganismus in einer Probe, wobei das Verfahren umfasst:
(a) Mischen der Probe oder eines Teils der Probe, von der vermutet wird, dass sie einen Mikroorganismus enthält, mit einem Kulturmedium (170) und Anwenden von Kulturbedingungen, die die Bildung mindestens eines flüchtigen Stoffwechselprodukts durch einen Mikroorganismus ermöglichen, wobei das Kulturmedium (170) in Schritt (a) mindestens ein schwefelhaltiges Molekül als Vorläufer enthält, um die Bildung von flüchtigem Sulfid durch den Mikroorganismus von Interesse zu ermöglichen;
(b) Nachweisen von Sulfid als Stoffwechselprodukt, falls erzeugt, durch eine Veränderung, die das Sulfid-Stoffwechselprodukt in einer elektrischen Eigenschaft oder einer magnetischen Eigenschaft und/oder einer elektromagnetischen Eigenschaft einer Gasdetektionsvorrichtung (130, 135), während die Gasdetektionsvorrichtung (130, 135) mit der Dampf-/Gasphase über dem Kulturmedium (170) oder benachbart dazu oder in Kommunikation damit in Kontakt steht, und
(c) Erhalten des Ergebnisses einer Änderung der elektrischen Eigenschaft oder der magnetischen Eigenschaft und/oder der elektromagnetischen Eigenschaft der Gasdetektionsvorrichtung (130, 135) als Anzeige auf die Abwesenheit oder das Vorhandensein oder die Population des Mikroorganismus im Kulturmedium (170) bzw. in der Probe,
wobei die Dauer des Mikrobenwachstums vom Beginn der Inkubation bis zum Auftreten und Nachweis des sulfidischen Stoffwechselprodukts gemessen und zur Berechnung der Abwesenheit oder Anwesenheit oder Population des Mikroorganismus im Testmedium (170) verwendet wird.

2. Verfahren nach Anspruch 1, wobei in Schritt (c) die Änderung der elektrischen oder magnetischen Eigenschaft und/oder der elektromagnetischen Eigenschaft ein Signal erzeugt, wenn ein Schwellenwert überschritten wird.

3. Verfahren nach Anspruch 1 oder 2, wobei der Nachweis in Schritt (b) eine teilweise oder vollständige Umwandlung in ein Metallsulfid oder ein anderes elektrisch leitfähiges Sulfid umfasst.

4. Verfahren nach einem der Ansprüche 1 bis 3, wobei Schritt (b) die Verwendung einer chemoresistiven Schicht umfasst, deren elektrischer Widerstand sich bei Kontakt mit gasförmigem oder dampfförmigem Sulfid ändert.

5. Verfahren nach einem der Ansprüche 1 bis 4, wobei das Erhalten des Ergebnisses in Schritt (c) die Übertragung eines Signals umfasst, das als Ergebnis der Änderung der elektrischen oder magnetischen und/oder elektromagnetischen Eigenschaft erzeugt wurde, an ein entferntes Instrument oder an einen entfernten Benutzer.

6. System zum Nachweis des Vorhandenseins oder zur Quantifizierung der Population eines Mikroorganismus in einer Probe, umfassend:
ein Kulturmedium (170), das die Erzeugung mindestens eines Sulfid-Stoffwechselprodukts durch einen Mikroorganismus ermöglicht;
eine Gasdetektionsvorrichtung (130, 135), die so angeordnet ist, dass sie mindestens ein von dem Mikroorganismus erzeugtes Stoffwechselprodukt durch Induzieren einer Änderung einer Eigenschaft der Gasdetektionsvorrichtung (130, 135) detektiert, wobei die geänderte Eigenschaft aus der Gruppe ausgewählt ist, die aus einer elektrischen Eigenschaft, einer magnetischen Eigenschaft und einer elektromagnetischen Eigenschaft besteht; und
ein Messinstrument, das so angeordnet ist, dass es eine Änderung der elektrischen oder der magnetischen und/oder der elektromagnetischen Eigenschaft der Gasdetektionsvorrichtung (130, 135) erfasst,
wobei die Gasdetektionsvorrichtung (130, 135) eingerichtet ist, um organisches oder anorganisches Sulfid in gasförmiger oder dampfförmiger Form zu detektieren, wobei ein von der Gasdetektionsvorrichtung (130, 135) detektiertes Stoffwechselprodukt zur chemischen Familie der Sulfide gehört,
wobei die Gasdetektionsvorrichtung (130, 135) mit der Dampf-/Gasphase über dem Kulturmedium (170) oder benachbart dazu oder in Kommunikation damit in Kontakt steht und ein Signal, erzeugt, wenn ein Schwellenwert überschritten wird, wodurch das Vorhandensein oder die Population des Mikroorganismus angezeigt wird, und
wobei das System so konfiguriert ist, dass die Dauer des mikrobiellen Wachstums vom Beginn der Inkubation bis zum Auftreten und Nachweis des sulfidhaltigen Stoffwechselprodukts gemessen und zur Berechnung der Abwesenheit oder Anwesenheit oder Population des Mikroorganismus im Testmedium (170) verwendet wird.

7. Das System nach Anspruch 6, das ferner eine Signalverarbeitungs- und Datenübertragungsbaugruppe umfasst, die von dem Messgerät empfangene Signale verwendet, um das Vorhandensein oder die Population des Mikroorganismus aus der Probe oder dem Teil der Probe zu bestimmen, und das Ergebnis über elektronische Mittel an einen entfernten Benutzer überträgt.

8. Das Verfahren nach einem der Ansprüche 1 bis 5 oder das System nach einem der Ansprüche 6 bis 7, wobei das nachzuweisende Stoffwechselprodukt während des mikrobiellen Wachstums in einem beliebigen Kopfraum (180) und/oder einem anderen Volumen, das an die Testkultur angrenzt oder mit dieser in Kommunikation steht, ansammeln kann, wobei der Kopfraum (180) oder das andere Volumen vorzugsweise über dem Medium (170) liegt, das für die Kultivierung von Mikroorganismen in einem versiegelten Behälter verwendet wird, oder mit diesem in Kommunikation steht.

9. Das Verfahren nach einem der Ansprüche 1 bis 5 oder das System nach einem der Ansprüche 6 bis 8, wobei der Nachweis von Sulfidsignalen anzeigt, dass die Testkultur einen Zustand der Bereitschaft für bestätigende biochemische oder mikrobiologische Tests erreicht hat.

10. Verfahren nach einem der Ansprüche 1 bis 5 oder System nach einem der Ansprüche 6 bis 9, wobei das zur Detektion von Sulfid verwendete aktive Detektionsmaterial eine Löslichkeitsproduktkonstante (Ksp) von weniger als 1x10⁻¹ bei 25 °C aufweist und/oder wobei die aktive Komponente des Sensors Kupfer(1)oxid ist.

11. Das Verfahren nach einem der Ansprüche 1 bis 5 oder das System nach einem der Ansprüche 6 bis 10, wobei die Reaktion der Gasdetektionsvorrichtung auf Sulfid die elektrischen oder magnetischen oder elektromagnetischen Eigenschaften einer drahtlosen Signalkomponente verändert oder eine drahtlose Signaleinrichtung (200, 215, 230) aktiviert, wodurch die Detektion oder Nichtdetektion von Sulfid durch drahtlose Übertragung an ein Lesegerät, einen Empfänger oder ein anderes elektronisches Gerät gemeldet wird.

## Revendications

1. Procédé pour détecter la présence ou quantifier la population d'un micro-organisme dans un échantillon, le procédé comprenant:
(a) le mélange de l'échantillon ou d'une partie de l'échantillon suspecté de contenir un micro-organisme avec un milieu de culture (170), et l'application de conditions de culture qui permettent la génération d'au moins un produit métabolique volatil par un micro-organisme, dans lequel le milieu de culture (170) dans l'étape (a) contient au moins une molécule soufrée en tant que précurseur pour permettre la génération de sulfure volatil par le micro-organisme d'intérêt;
(b) détecter le sulfure en tant que produit métabolique, s'il est généré, par un changement que ledit produit métabolique sulfure induit dans une propriété électrique ou une propriété magnétique et/ou une propriété électromagnétique d'un dispositif de détection de gaz (130, 135), tandis que le dispositif de détection de gaz (130, 135) est en contact avec la phase vapeur/gaz au-dessus ou à proximité ou en communication avec le milieu de culture (170), et
(c) obtenir le résultat d'un changement dans la propriété électrique ou la propriété magnétique et/ou la propriété électromagnétique du dispositif de détection de gaz (130, 135) comme indication de l'absence ou de la présence ou de la population du micro-organisme dans le milieu de culture (170), ou respectivement dans l'échantillon,
dans lequel la durée de la croissance microbienne, depuis le début de l'incubation jusqu'à l'apparition et la détection du produit métabolique sulfuré, est mesurée et utilisée pour calculer l'absence ou la présence ou la population du micro-organisme dans le milieu d'essai (170).

2. Procédé selon la revendication 1, dans lequel, à l'étape (c), le changement de la propriété électrique ou magnétique et/ou de la propriété électromagnétique génère un signal lorsqu'une valeur seuil est franchie.

3. Procédé selon la revendication 1 ou 2, dans lequel la détection à l'étape (b) implique une conversion partielle ou complète en un sulfure métallique ou autre sulfure électriquement conducteur.

4. Procédé selon l'une quelconque des revendications 1 à 3, dans lequel l'étape (b) implique l'utilisation d'un film chimiorésistif dont la résistance électrique est modifiée lorsqu'il est en contact avec un sulfure en phase gazeuse ou vaporeuse.

5. Procédé selon l'une quelconque des revendications 1 à 4, dans lequel l'obtention du résultat à l'étape (c) implique le transfert d'un signal, qui a été généré à la suite de la modification de la propriété électrique ou magnétique et/ou électromagnétique, vers un instrument distant ou vers un utilisateur distant.

6. Système pour détecter la présence ou quantifier la population d'un micro-organisme dans un échantillon, comprenant:
un milieu de culture (170) qui permet la génération d'au moins un produit métabolique de sulfure par un micro-organisme;
un dispositif de détection de gaz (130, 135) agencé pour détecter au moins un produit métabolique généré par le micro-organisme en induisant une modification d'une propriété du dispositif de détection de gaz (130, 135), la propriété modifiée étant choisie parmi le groupe constitué d'une propriété électrique, d'une propriété magnétique et d'une propriété électromagnétique; et
un instrument de mesure agencé pour détecter un changement dans la propriété électrique ou magnétique et/ou électromagnétique dudit dispositif de détection de gaz (130, 135),
dans lequel le dispositif de détection de gaz (130, 135) est agencé pour détecter un sulfure organique ou inorganique sous forme gazeuse ou vaporeuse, dans lequel un produit métabolique détecté par le dispositif de détection de gaz (130, 135) appartient à la famille chimique des sulfures,
dans lequel le dispositif de détection de gaz (130, 135) est en contact avec la phase vapeur/gaz au-dessus ou à proximité du milieu de culture (170) ou en communication avec celui-ci et génère un signal d' lorsqu'une valeur seuil est dépassée, indiquant ainsi la présence ou la population du micro-organisme, et
dans lequel le système est configuré de telle sorte que la durée de la croissance microbienne, depuis le début de l'incubation jusqu'à l'apparition et la détection du produit métabolique du sulfure, est mesurée et utilisée pour calculer l'absence ou la présence ou la population du micro-organisme dans le milieu d'essai (170).

7. Le système selon la revendication 6, comprenant en outre un ensemble de traitement de signaux et de transmission de données qui utilise les signaux reçus de l'instrument de mesure pour déterminer la présence ou la population du micro-organisme dérivé de l'échantillon ou de la partie de l'échantillon, et transmet le résultat par des moyens électroniques à un utilisateur distant.

8. Procédé selon l'une quelconque des revendications 1 à 5, ou système selon l'une quelconque des revendications 6 à 7, dans lequel le produit métabolique à détecter est autorisé, au cours de la croissance microbienne, à s'accumuler dans un espace libre (180) et/ou tout autre volume adjacent ou en communication avec la culture test, de préférence dans lequel l'espace libre (180) ou tout autre volume est situé au-dessus ou en communication avec le milieu (170) utilisé pour la culture des micro-organismes à l'intérieur d'un récipient scellé.

9. Procédé selon l'une quelconque des revendications 1 à 5, ou système selon l'une quelconque des revendications 6 à 8, dans lequel la détection de sulfure indique que la culture d'essai a atteint un état de préparation pour des tests biochimiques ou microbiologiques de confirmation.

10. Procédé selon l'une quelconque des revendications 1 à 5, ou système selon l'une quelconque des revendications 6 à 9, dans lequel le matériau de détection actif utilisé pour la détection du sulfure a une constante de solubilité (Ksp) inférieure à 1x10⁻¹ à 25 °C, et/ou dans lequel le composant actif du capteur est l'oxyde de cuivre(I).

11. Procédé selon l'une quelconque des revendications 1 à 5, ou système selon l'une quelconque des revendications 6 à 10, dans lequel la réponse du dispositif de détection de gaz au sulfure modifie les propriétés électriques, magnétiques ou électromagnétiques d'un composant de signalisation sans fil, ou active un dispositif de signalisation sans fil (200, 215, 230), annonçant ainsi la détection ou la non-détection de sulfure à un lecteur, un récepteur ou un autre dispositif électronique par transmission sans fil.
